(19) **Europäisches Patentamt European Patent Office Office européen des brevets**

(11) **EP 4 431 163 B1**

(12) **EUROPEAN PATENT SPECIFICATION**

(45) Date of publication and mention
of the grant of the patent:
**08.07.2026 Bulletin 2026/28**

(21) Application number: **22888805.3**

(22) Date of filing: **16.05.2022**

(51) International Patent Classification (IPC):
*A63B 22/04* (2006.01)    *G16H 20/30* (2018.01)
*A61H 39/04* (2006.01)    *A61H 1/02* (2006.01)
*A63B 21/005* (2006.01)   *A63B 23/035* (2006.01)
*A63B 23/04* (2006.01)    *A63B 71/06* (2006.01)
*A63B 22/00* (2006.01)

(52) Cooperative Patent Classification (CPC):
**A61H 1/0244; A61H 1/024; A61H 1/0255;
A63B 21/0058; A63B 23/03508; A63B 23/0482;
A63B 71/0622; G16H 20/30;** A61H 2201/0161;
A61H 2201/0192; A61H 2201/1635;
A61H 2201/1642; A61H 2201/1676;
A61H 2203/0406; A63B 69/0057;         (Cont.)

(86) International application number:
**PCT/CN2022/092912**

(87) International publication number:
**WO 2023/077768 (11.05.2023 Gazette 2023/19)**

(54) **KNEE OSTEOARTHRITIS TREATMENT AND REHABILITATION EQUIPMENT BASED ON KOAPT**

KNIEOSTEOARTHRITISBEHANDLUNG UND REHABILITATIONSAUSRÜSTUNG AUF KOAPT-BASIS

ÉQUIPEMENT DE TRAITEMENT ET DE RÉÉDUCATION D'ARTHROSE DU GENOU FONDÉ SUR KOAPT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.11.2021 CN 202111313071**

(43) Date of publication of application:
**18.09.2024 Bulletin 2024/38**

(73) Proprietor: **Wuhan Kede Medical Instrument Co., Ltd.**
**Wuhan, Hubei 430000 (CN)**

(72) Inventors:
• **SONG, Jiuhong**
**Wuhan, Hubei 430000 (CN)**
• **LIU, Xinting**
**Wuhan, Hubei 430000 (CN)**
• **ZHANG, Guosheng**
**Wuhan, Hubei 430000 (CN)**

(74) Representative: **Lin Chien, Mon-Yin
Gloria Fuertes 1, 2° D
28342 Valdemoro Madrid (ES)**

(56) References cited:
CN-A- 105 832 496      CN-A- 106 726 358
CN-A- 107 692 964      CN-A- 108 245 840
CN-A- 109 481 237      CN-A- 110 013 648
CN-A- 112 316 378      CN-A- 113 952 677
US-A1- 2008 234 113    US-B2- 9 314 393

(52) Cooperative Patent Classification (CPC): (Cont.)
A63B 2022/0094; A63B 2023/0452;
A63B 2071/0655; A63B 2071/0658;
A63B 2208/0209

## Description

### TECHNICAL FIELD

The present invention relates to the technical fields of clinical medical device and home rehabilitation exercises, specifically referring to a KOAPT-therapy-based treatment and rehabilitation device for degenerative knee joint diseases.

### BACKGROUND

The world has entered an aging society, with the proportion of the elderly population increasing year by year. Degenerative knee joint diseases have become one of the major disabling illnesses globally, seriously affecting the quality of life of the elderly. As of the end of 2018, statistics show that over 60% of people aged 65 and above suffer from knee joint pain, and the number of knee osteoarthritis patients has exceeded 150 million. China is one of the countries with the highest number of knee osteoarthritis patients.

The knee joint is the most complex weight-bearing joint in the human body, and it is also one of the joints most susceptible to injury. Knee joint disorders usually refer to a series of pathological and physiological changes within the joint cavity, including arthritis, osteoarthritis, synovitis, patellar softening, meniscus injuries, subchondral bone sclerosis, subchondral bone deformation of various kinds (becoming, e.g. convex concave-surfaced, osteoporotic, collapsed or cystically hollowed), which result from any of the various factors such as instability in the lower limb biomechanical alignment, disuse atrophy of the lower limb, prolonged incorrect working or sitting postures, inadequate blood supply to the lower limbs, joint cavity infections, primary and metastatic tumors within the joint cavity, osteoporosis by endocrine disorders, and high-energy external injuries. Knee osteoarthritis (KOA), also known as proliferative knee arthritis or senile knee arthritis (commonly known as "cold legs" in Chinese), is the most common chronic degenerative joint disease. The earliest and most significant pathological changes occur in the cartilage, followed by subchondral bone cystic lesions, formation of osteophytes at the joint margins, and eventually destruction and deformity of the normal joint surface,

With the arrival of an aging society in China, both the incidence and total numbers of patients with knee osteoarthritis (KOA) are showing a significant increasing trend. KOA patients suffer great physical pain: they experience significant agony and weakness in the knees when going up and down stairs, and feel fatigued and uncomfortable when walking for a long time. Severe patients may even be unable to stand alone and must rely on support to stand. Long-term sufferers not only have a significantly reduced quality of life, but also inevitably suffer from psychological trauma.

Modern clinical medicine has proposed different treatment plans for KOA patients at different stages of the Kellgren-Lawrence (K-L) grading. In terms of drug treatment, both locally acting analgesics and systemic analgesics widely used in clinical practice have limitations. They cannot fundamentally address the pathological and physiological changes of KOA, nor can they prevent further deterioration. They can only temporarily relieve pain for a very limited time, and cannot address issues such as muscle weakness, muscle atrophy, and organic lesions such as meniscal cysts, meniscal tears, cartilage fractures, tears of anterior and posterior cruciate ligaments, and lower limb biomechanical instability. Long-term use also carries a series of potential adverse reactions. Total Knee Arthroplasty (TKA, UKA) is another major category of treatment applied to KOA patients at the fourth stage of the K-L grading. However, this type of surgery involves destructive reconstruction and causes significant damage, resulting in low patient acceptance. Moreover, after knee replacement surgery, patients' knee muscle strength and lower limb proprioception cannot meet the demands of normal daily activities. Particularly, as a result of the fact that the entire lower limb proprioception system has been compromised, patients face a continuous and indefinite risk of falling in the future. Other treatment options such as weight reduction, orthotic devices, intra-articular hyaluronic acid sodium injections, autologous platelet-rich plasma (PRP) injections, intra-articular ozone injections, and so on, have limited effectiveness, only providing short-term control of local symptoms and limited slowing of functional decline. In the long term and from a systemic perspective, these treatments have significant limitations and cannot fundamentally resolve knee joint pain, provide sufficient muscle support around the knee joint, restore good proprioception for patients in the course of voluntary motion, and achieve efficient painless and damage-free systemic treatment at any time and any place.

In the "Clinical Practice Guidelines for the Management of Osteoarthritis Pain in China (Chinese Journal of Orthopedics, Edition 2020)", exercise therapy has been designated as a 1A (the most strongly recommended level) choice for treating Knee Osteoarthritis (KOA). As early as the Western Han Dynasty in China, the "Yinshu" (Book of Remedies) proposed a prescription for "Knee Pain Guidance": "For knee pain, if the right knee hurts, clench the left hand, and swing the right foot inward, repeating a thousand times; if the left knee hurts, clench the right hand, and swing the left foot inward, repeating a thousand times. Hook the left toes with the left hand, pull them back, repeating ten times. Then, support with the left hand clenched, hook the right toes with the right hand, pull them back, repeating ten times." This instructs patients to perform leg swinging exercises, promoting leg muscle contraction and relaxation to enhance leg blood circulation. The generation of heat through leg swinging helps alleviate the symptoms of cold and pain in the lower leg, reflecting this practice's underlying principle of "activating blood circulation, resolving stasis, warming the Jing Mai (a definition in traditional Chinese medicine, means passages through which vital energy circulates) and dispelling cold". Treating knee pain through the Knee Pain Guidance is an effective and classic therapy for degenerative knee joint conditions. Leg swinging can effectively treat knee joint pain, but in modern clinical applications, most KOA patients do not fundamentally benefit from it for the reasons listed as follows:

From a psychological perspective: Due to the monotony of the exercise, patients feel mentally restless and cannot consistently adhere to the Knee Pain Guidance exercise.

2. From a physical standpoint: Single-leg support and swinging, with over 1000 repetitions on each side daily, lead to physiological discomfort and resistance in the initial stages of treatment, making it difficult to form habitual tolerance of and cultivate daily compliance with the requirements of the Knee Pain Guidance, ultimately failing the treatment.

3. From the perspective of modern evidence-based medicine: Although Knee Pain Guidance has been clinically recognized by patients for two thousand years, its scientific fundamentals are not yet clear, and nor are the movement standards well-defined. Knee Pain Guidance cannot convincingly become a standard clinical treatment for KOA for modern clinicians.

Currently, there is no treatment method or product in the world that fundamentally resolves the pain of KOA that can comprehensively surpass Knee Pain Guidance in terms of cost-effectiveness and in respect of pathophysiological fundamentals. The researchers of this invention have long committed to the study of KOA diagnosis and treatment methods, proposing the K-KOA (Kill Knee Osteoarthritis, KKOA) staged therapy based on the K-L grading and the modern standard leg-pendulum-motion therapy for KOA at specific stages (Knee Osteoarthritis Pendulum Therapy, KOAPT). There are similarities and differences between KOAPT and the Knee Pain Guidance. Both aim to treat KOA through leg swinging. However, compared to the rough description of treatment steps of the Knee Pain Guidance, KOAPT is a more precise, standardized, and continuous rehabilitation treatment method that addresses the root causes of KOA, designed to remove both pain symptoms and weakness of the muscles around the knee joint. KOAPT can reduce the intra-articular pressure of the knee joint, thereby promoting intra-articular blood circulation, synovial fluid circulation, and enhancing the metabolism of intra- and peri-articular tissues. It can also efficiently strengthen the anterior and posterior leg muscles and significantly improve the sensitivity of the lower limb proprioceptors.

The inventor of this device is the person proper who first made the relevant scientific discovery, which will be published for the first time in the UK medical journal 'Trails'.

Therefore, a treatment and rehabilitation device like this invention is urgently needed to help bring the curing potentials of KOAPT into reality.

US 2008/0234113 A1 disclose that a gait rehabilitation methods and apparatuses, to allow the patient to exercise without bearing the patient's full weight on the rehabilitating limbs. As strength and control return to the patient, weight can be gradually added until the patient is bearing full weight. In paragraph 0106, the sensors may optionally monitor the patient's position, acceleration, force, and/or velocity. The data output by the sensors is analyzed and thus, anomalies in the gait are detected, either by the controller or by a healthcare professional. From this data analysis of the gait, it can be determined where improvements need to be made. Optionally, the controller gives advisory instructions on how to improve the patient's gait.

## SUMMARY

To overcome the shortcomings of existing technologies, this invention discloses a treatment and rehabilitation device for knee osteoarthritis based on the therapy, which urges patients to exercise the KOAPT in conformity with their respective schedules and keep their leg swinging movements up to standard. This helps KOA patients overcome the physiological and psychological pains and resistance to KOAPT exercises during the treatment process, and eventually addresses the root causes of the pains experienced by KOA patients through continuous and repeated KOAPT exercises, bringing hopes of a painless life to KOA patients worldwide.

To achieve the above purpose, the treatment and rehabilitation device for KOA designed by this invention is novel in that set out in the appended set of claims.

The KOA (Knee Osteoarthritis) disease is classified in imaging examinations pursuant to the KL (Kellgren-Lawrence, K-L) Grading System. Under this System, the severity of the condition of a KOA patient is identified and an individual plan is worked out accordingly for his/her treatment.

The grades of the K-L Grading System is as follows:

1. Grade 0: Completely normal joint.

2.Grade I: Slight osteophyte formation in the joint, but no significant narrowing of the joint space.

3.Grade II: Narrowing of the joint space and osteophyte formation.

4.Grade III: Moderate osteophyte formation, clear narrowing of the joint space, sclerosis of the bone under the cartilage, or joint deformities such as varus or valgus deformities.

5.Grade IV: Extensive bone spurs, severe narrowing of the joint space, obvious bone sclerosis, and very apparent joint deformities.

Common physiological conditions in KOA patients include heavy weight, atrophy and weakness of the flexor and extensor muscle groups around the knee joint (such as quadriceps, hamstring muscles...), and an age usually over 45 years old.

Common symptoms in KOA patients include knee joint movement pain, instability when walking on flat ground, weakness when ascending or descending stairs, and compensatory deformities in body shape.

Pathological changes in the body of KOA patients include degeneration and deformation or even rupture of knee joint cartilage, narrowing of the knee joint space, uneven surface of the subchondral bone, formation of osteophytes and bone spurs, increase in inflammatory factors in synovial fluid within the joint cavity, fragments of cartilage and subchondral bone, even depositing of fragmented joint tissues in the joint cavity.

The present invention is developed based on the various internal and external conditions and the unique physiological and pathological reasons of KOA patients at different stages of the disease. It aims to guide, supervise and drive KOA patients to perform KOAPT exercises, and to advise them in due course to perform right and up to scientific standards. Simultaneously, the KOAPT machine-guided KOAPT exercise treatment, in conjunction with other specific KOA treatment methods (such as arthroscopic lavage, debridement, and repair; lower limb alignment correction surgery; postoperative lower limb muscle strength rehabilitation training for UKA, TKA...), forms a treatment plan, namely the KKOA (Kill Knee Osteoarthritis, KKOA) treatment plan, which is rightly graded, integrates different-measures, and covers all KOA stages.

This KOAPT machine is invented on the basis of the prescribed movements of the "Knee Pain Guidance", and rigorous pathophysiologic and rehabilitation-kinesiological rationales, and through the analysis of results of both clinical research into a large group of KOA patients and animal experiments. The principles of the invention include:

1. During KOAPT exercises, the pressure in the knee joint cavity decreases, leading to accelerated blood circulation through and over relevant tissues in the knee joint cavity. This, in turn, increases the efficiency of clearing inflammatory pain factors within the joint cavity and enhances tissue repair capabilities. The relevant tendons and ligaments located inside or outside the knee joint that had contracted gradually loosen, increasing the joint space. The pressure on the subchondral bone decreases and, because there are abundant nerve endings on the subchondral bone, a significant reduction in KOA pain is reported.

2. KOAPT exercises can effectively and progressively increase the strength of the surrounding flexor and extensor muscle groups of the knee joint, which are necessary for normal movement, while minimizing the pains experienced by KOA patients, until the muscle strength necessary for normal daily activities are regained.

3. Performing leg swings more than a thousand times each day, consistently for over a year, has a positive effect on reducing the weight burden. Conversely, weight gain and lack of proper lower limb muscle exercise are the main contributing factors to the occurrence and progression of KOA. As weight increases and the knee pains worsen, the patient will become more reluctant and resistant to do knee joint movements of any kind, leading to a vicious cycle. However, KOAPT under the guidance of the KOAPT machine serve as a brilliant antidote to it.

According to modern evidence-based medical theory and clinical observations over the past five years, the Knee Pain Guidance does not scientifically specify the duration of continuous leg support. However, prolonged single-leg support may cause ischemic and compressive lesions in the subchondral bone of the supporting leg, resulting in increased pain and accelerated functional degeneration. Additionally, the range and frequency of leg swings have not been scientifically validated and limited to their maximum and minimum values.

Description and explanation of individual differences in body gravity line during the overall swinging process, head and neck angles, arm support point height, patient strength, height, weight, age, and other factors have not reached the level where common fundamentals of mutual benefit and non-contradiction while functioning parallel meet practices of individualized diagnosis and treatment in dialectical unification. Compared to existing technologies, the beneficial technical effects of this invention are evident in:

(1) If KOA patients do not engage in the Knee Pain Guidance exercises for two consecutive weeks, the pressure within the joint cavity will increase, leading to reduced blood and synovial fluid circulation in the joint tissues, resulting in decreased synovial fluid and spells of pain. This invention enables users to engage in KOAPT exercises continuously.

(2) This invention utilizes a CCR system to supervise, correct, and provide feedback on KOAPT exercise postures, to achieve the purposes of standardizing correct and comfortable exercise postures and, in a proactive mode, assisting patients in overcoming physical pains and psychological resistance experienced during exercise.

(3) This invention designs information feedback from multiple dimensions, with a view to addressing the problem of restlessness caused by monotonous exercise, enabling patients to engage in the Knee Pain Guidance exercise with attention and composure, and freeing their hands so that they can work and communicate with others simultaneously.

(4) This invention is embodied in models of different external features to accommodate conditions of different environments such as medical institutions, home, and travel stays, and all models come with portability, reliability, convenience and durability.

**BRIEF DESCRIPTION OF THE DRAWINGS**

Figure 1: Schematic diagram of the system structure of the device for treating and rehabilitating knee osteoarthritis, the device is designed based on knee osteoarthritis pendulum therapy.

Figure 2: System architecture diagram of the KOAPT computer program.

Figure 3 and Figure 4: Workflow diagrams of the treatment process control module.

Figure 5: Right side view of the device in operation.

Figure 6: Assembly structure diagram of the multi-functional support frame.

Figure 7: Exploded view of the parts of the multi-functional support frame.

Figure 8: Left side view of the folding leg swing mechanism in operation.

Figure 9: Assembly structure diagram of the lower leg length adjustment frame.

Figure 10: Schematic diagram of the combination principle of the folding leg swing mechanism.

Figure 11: Schematic diagram of the principles and structure of the dual-drive version of the folding leg swing mechanism.

Figure 12: Schematic diagram of the folding, stretching and retracting principle of the folding leg swing mechanism and the multi-functional support adjuster.

Figure 13: Schematic diagram of the status of the folding leg swing mechanism when folded.

Figure 14: Schematic diagram of the principle and structure of the dual-drive version of the folding leg swing mechanism with a flip-folding seat.

Figure 15: Schematic diagram of the principle and structure of the folding leg swing mechanism with a flip-folding seat.

Figure 16: Left side view of the installation position of the photoelectric sensor.

Figure 17: Top view of the installation position of the photoelectric sensor.

Figure 18: Schematic diagram of the installation position of the TOF sensor.

Figure 19: Schematic diagram of the camera installation position.

Figure 20: Schematic diagram of the installation position of the distance sensor and the angle calculation principle.

Figure 21: Schematic diagram of the working principle of the multi-functional support adjuster.

Figure 22: Structural schematic diagram of the terminal support frame.

Folding leg swing mechanism 1, Multi-functional support frame 2, Multi-functional support adjuster 3, Terminal support frame 4, Display 5, First sensor group 61, Second sensor group 62, Third sensor group 63, Fourth sensor group 64, Flip-folding seat 10;

the Folding leg swing mechanism 1 includes: Pivot 1a, Housing 11, Pivot hole 11a, Housing linkage-fastening hole 11b, Foot pedal 12, Massage protrusion 12a, Anti-slip protrusions 12b, Folding telescopic frame 13, Motor 14, Swing angle frame 15, Lower leg length adjustment frame 16, Gear case 17, Coupling 18, locking handle 19, Pedal surface 121, Telescopic rod 131, Folding frame 132, Linkage pivot 132a, Housing linkage 133, First rod body 151, Second rod body 152, First reinforcing flange 153, U-shaped frame 161, First securing frame 162a, Second securing frame 162b, Second slider 163a, Third slider 163b, Guide rod 164, First slider 165, Footrest plate 165a, Plate linkage 165b, Locking mechanism 165c, First guide hole 164a;

the multifunctional support frame 2 includes: support base 21, support rod 22, Cam locking ring 23, support adjustment rod 24, support bracket locking ring 25, elbow and armpit dual-use support bracket 26, guide rail mounting hole 211, support rod fixing hole 212, third rod 241, fourth rod 242, second reinforcing flange 243;

the multifunctional support adjuster 3 includes: first guide rail 31, second guide rail 32;

the terminal support frame 4 includes: straight frame 41, rotating U-shaped frame 42, remote control holder 43, all-direction adjustment bracket 44.

## DETAILED DESCRIPTION OF THE EMBODIMENTS

The following is a detailed description of the present invention in conjunction with the accompanying drawings and specific embodiment cases.

Definition

KOAPT: Knee Osteoarthritis Pendulum Therapy, the therapy requires users to perform leg swinging movements as a treatment;

Graduated Treatment Models: match the user to a treatment model in accordance to his personal information input or the doctor's advice on exercise; The grading of treatment for a disease according to the severity of the patient is familiar to those skilled in the art. Moreover, the multiple preset treatment options included in a graduated treatment model may vary depending on the settler. In the following embodiment, the graduated treatment models are set by the inventor, but can be replaced by other KOA treatment models in the prior art.

CCR: Correct and Comfortable Relationship body data, refers to the relationship between correct and comfortable body postures subject to KOAPT requirements and body movement forms; in the present invention, the body data includes elbow support point height, reduction of body weight on the supporting leg, trunk upright status value, and leg upright state value;

Elbow Support Point Height: height from the surface of foot pedals to the elbow support point, there are two forms of the user's posture, one is the user has two arms on each side of the body as a form of support; the other is the user places two arms in front of the chest as a form of support;

The User's CCR Indicative Value $y$: the value indicates whether the user's Correct and Comfortable Relationship body data correct; 1 is for correct and 0 is for incorrect;

Trunk Upright Indicative Value $k1$: the value indicates whether the user's trunk upright, 1 is for correct and 0 is for incorrect;

Leg Upright Indicative Value $k2$: the value indicates whether the user's leg upright, 1 is for correct and 0 is for incorrect;

Supporting Leg Load Reduction Indicative Value $k3$: the value indicates whether the user's leg load reduction, 1 is for correct and 0 is for incorrect;

Comfort Indicative Value $k4$: the value indicates whether the user's posture comfort, 1 is for correct and 0 is for incorrect;

Redundancy Indicative Value $k5$: with a default value of 0.

The user's leg pivot: the 'pivot' in this phrase corresponds to the greater trochanter of the user's leg. the pivot point of the leg swinging movements is only the greater trochanter.

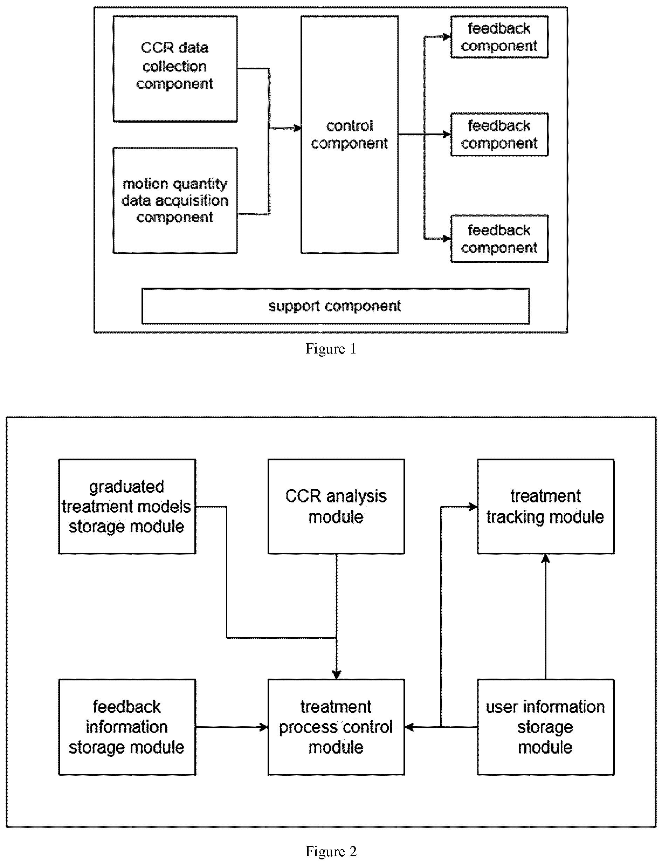

The device for knee joint degenerative diseases based on KOAPT proposed by the present invention, as shown in Figure 1, includes the CCR data collection component, motion quantity collection component, support component, control component, and feedback component.

Support component: used to provide trunk support and/or waist support and/or elbow support for the user. The support component is designed with different external features to fit different users and scenarios. Though the present embodiment proposes a folding component structure, it doesn't limit other functions of the support component. Mechanisms that provide trunk support and/or waist support and/or elbow support for the user while the system is in operation are all included in the support component described in the present invention.

CCR data collection component: Installed on the support component, it is used to collect CCR data of the user on KOAPT on this device. CCR, short for Correct and Comfortable Relationship, refers to the relationship between correct and comfortable body postures subject to KOAPT requirements and body movement forms. CCR data includes user height, distance between elbow support point and stepping point, height of support on both sides, height of chest support, user weight, weight on support of both sides, weight on support of chest, upright status of trunk, upright status of legs, etc.

Motion quantity collection component: Installed on the support component, it is used to real-time collect the motion quantity data of leg swinging of the user on this device. Motion quantity data includes the number of leg swings, movement frequency, and movement amplitude of motion.

Control component: Used to execute the KOAPT computer program deployed in the control module, real-time collect the CCR data of the user on this device, real-time determine whether the comfortable support point height, supporting leg load reduction, trunk upright status value, and upright leg status value during the user's exercise meet the preset CCR value; And, when the user's CCR doesn't meet the KOAPT requirements, it outputs feedback instructions to control the feedback component to communicate corresponding information to the user, with a view to correcting his/her movement posture; It also real-time collects the user's motion quantity data to guide the user to complete the treatment and rehabilitation exercise according to the system of graduated treatment models.

The control component includes a control device, communication module, power module, etc.; and the communication module can use transmission methods such as WIFI, wireless Bluetooth, etc. The control device can be set on the support component, interact with the user through the human-computer interactive display screen, or be remotely set on the server to communicate with the user through the mobile device, or the server directly send control instructions to the feedback component mounted on the support component.

The feedback component: used to transmit audio and/or video and/or tactile signals to the user according to instructions of the control component. The part of the feedback component which does this can be a display screen, speaker, massage protrusions, motor, etc., installed on the support component; It transmits visual or audio information, or does it through controlling the vibration of massage protrusions or driving the support component to move in a particular manner. The feedback component can also be integrated into a mobile device, transmitting visual, audio or other types of information etc., according to instructions of the control component.

The structure diagram of the KOAPT computer program, shown in Figure 2, presents all its component modules, namely the graded model storage module, CCR analysis module, treatment process control module, treatment tracking module, feedback information storage module and user information storage module, and their structural relations.

Graduated treatment models storage module: used to store the graduated treatment models;

CCR analysis Module: used to input the real-time collected CCR data into the CCR analysis model, to real-time determine the user's posture information, and to output CCR judgment instructions; When the user's CCR is found to be unsatisfactory, it simultaneously outputs an unsatisfactory flag;

The treatment process control module: is used to call the feedback instructions according to the CCR data judgment instructions; the treatment process control module matches the motion quantity data collected by the motion quantity data collection component with values in the graduated treatment models, and according to matching results, calls the feedback instructions; the feedback instructions are stored in the feedback information storage module;

The treatment tracking module: used to monitor and control the completion progress of the user's treatment process and to call the corresponding feedback instructions in the feedback information storage module; the feedback information storage module includes count standard feedback information, CCR standard feedback information, incentive feedback information, separation alarm information, and media information;

User information storage module: used to store the user's personal information, CCR data, and motion quantity data.

The graduated treatment model includes Level $X1$, Level $X2$ and Level $X3$ treatment models. Each graduated treatment model has preset multiple parameters, including graduated period, times of supporting the body on one leg $V$, movement amplitude $\theta$, movement frequency $\omega$, leg assistance force $F$, movement quantity $C$, necessary supporting leg rest time $t$, and supporting leg load reduction $\Delta a$. The parameters of the graduated treatment model are shown in Table 1. For example, the $X1$ level treatment model is suitable for individuals with a BMI over 28 who are unwilling to exercise voluntarily, experiencing severe knee pain and diagnosed K-L grade 3 or above. For these $X1$ level users, within 0-8 weeks, additional force is applied to the swinging leg; the times of supporting the body on one leg $V$ is 50; the movement amplitude $\theta$ is from -30 to +30°; the movement frequency $\omega$ is 30 times per minute; the support component's motor provides leg assistance force $F$; the motion quantity $C$ is 500 times; the necessary supporting leg rest time $t$ is at least 1 minute; and the supporting leg load reduction is $\geq$20%, with the user's body weight as the reference value.
Graduated treatment models can match the user to a treatment model in accordance to his personal information input or the doctor's advice on exercise. Table 1 shows the clinical corresponding indices of graduated treatment models needed to assist treatment.

Table 1

| Treatment Model | Graduated Period | Leg Assistance Force $F$ | Rehabilitation Goal | Moveme nt Amplitud e$\theta$ | Swinging times of supporting the body on one leg $V$ | Necessary supporting leg rest time $t$ |
|---|---|---|---|---|---|---|
| $X1$ Level Treatment Model | 0-8 weeks | Additional Force | Adaptive exercise | >±30° | 50 | >1min |
| | 9-12 weeks | | Pain alleviation | -30~+45° | 50-60 | >1min |
| | | | | | | >1min |
| $X2$ Level Treatment Model | 13-16 weeks | Active Exer-cise | Pain elimination, tis-sue regrowth initiated | >±30° | 50 | >1min |
| | 17-20 weeks | | | >±30° | 50 | >1min |
| | | | | | | >1min |
| | 21-24 weeks | | tissue regrowth Maintenance exer-cise: quadriceps, bi-ceps and hamstrings | -30~+45° | 80 | >1min |
| | 25-52 weeks | | | | 100 | >1min |
| $X3$ Level Treatment Model | 53-104 weeks | Resistance (adjustable) | Degenerative changes inhibited, Strengthened muscle exercise: quadriceps, biceps and hamstrings | -30~+45° | 100 | >1min |

(continued)

| Treatment Model | Graduated Period | Movement Quantity $C$ | Supporting leg Load Reduction $\Delta a$ | Duration | Target Population |
|---|---|---|---|---|---|
| $X$1 Level Treatment Model | 0-8 weeks | 500 | ≥20% | Need to be done daily, recommended to be completed at once; if not possible, done in two or three sessions | Obese with BMI≥28, unwilling to exercise, moderate to severe pain, K-L grade 3 and above |
| | 9-12 weeks | 500 | ≥20% | | |
| $X$2 Level Treatment Model | 13-16 weeks | 500 | ≥10% | Need to be done daily, recommended to be completed at once | Normal weight, having some exercise habits and capabilities, mild pain or no pain, K-L grade below 3 having needs of enhanced exercise |
| | 17-20 weeks | 500 | ≥10% | | |
| | 21-24 weeks | 800 | ≥10% | Need to be done daily, recommended to be completed at once | |
| | 25-52 weeks | 1000 | 0~5% | | |
| $X$3 Level Treatment Model | 53 -104 weeks | 1000-1200 | 0~5% | Need to be done daily, recommended to be completed at once | |

Generally, KOA patients can complete smoothly the KOAPT exercise within the swinging angle absolute-value range of 60° (±30°) or above. In cases where patients suffer muscle atrophy, decreased muscle strength and obesity, we found in clinical trials that if the KOAPT exercise angle range is 60° (±30) or below, the efficiency of improving the strength of the knee flexors and extensors is too low.

Additionally, only negligible decrease of the pressure within the joint cavity is recorded in experiments, i.e., an average of only 5mm water column. The decrease in pressure is so small that it leads to little increase in blood circulation in and around tissues within the joint cavity, and very low efficiency in loosening contracted connecting tissues (such as the anterior and posterior cruciate ligaments, medial and lateral collateral ligaments, patellar tendon, etc.)

However, when the absolute-value swinging angle of the patient on KOAPT exercise is above 60°(±30°) , the average pressure decrease in the knee joint cavity is above 15mm water column. It is evident that when the absolute-value swinging angle range is above 60° (±30°), the KOAPT has significant positive effect on pain relief, tissue regrowth, and improvement of muscle strength for KOA patients.

During the swinging exercise, the load on the supporting leg of the KOA patient is reduced for more than 20% (which is also one of the focuses of this invention). The reduction directly leads to a decrease in the pressure in the knee joint cavity of. Its clinical significance has been, through statistical analysis of treatment data, effectively verified in traditional KOA exercise therapies, such as swimming, supine pedaling, knee pain traction, the statistical treatment effects. Notwithstanding, KOAPT is the simplest, most comfortable, efficient, and sustainable among all these exercises.

The pressure in the knee joint cavity will increase as the body weight increases, thereby exacerbating degenerative changes in the knee cartilage and subchondral bone. We found that, during KOAPT exercise, the pressure borne by the supporting leg joint reaches four times that of the normal state. The number of continuous swinging cycles of KOAPT exercise should be limited within a framework of graduated levels on the basis of the K-L grading, real-time weight and muscle strength of the patient. For example, in the first level of KOAPT, 0-8 weeks of continuous swinging on one leg for 50 cycles each time did not cause any discomfort in the supporting leg. Conversely, we found that when it is raised to 100 cycles or above, patients experienced dull pain in the knee joint of the supporting leg and intermittent limping in daily life.

To achieve the treatment goals fully, KOAPT must be implemented in a way that satisfies truly the requirements of both the orthopedic medicine and the social surroundings in which it is performed. It is subject to this condition that the comprehensive systemic list of KOAPT indices of exercise positions and quantities has been developed.

CCR analysis module: used to input real-time CCR data into the analysis model, to make real-time judgments about user postures, and to output CCR judgment instructions. CCR adjustment: The user stands on this device, input personal information (gender, age, height, weight, facial information, K-L grading, pain indicators, doctor's directions about exercise, self-appointed supervisor's contact information, etc.); The control system imports the user-specific graduated treatment model, measures the user's height, weight (or input), calculates elbow height and thigh and calf lengths based on the height and weight, and automatically adjusts the elbow support point height and thigh and calf support lengths; The user confirms or makes minor adjustments and stores the settings.

The CCR analysis model is represented by the equation $y = k1 + k2 + k3 + k4 + k5$, where y is the user's CCR indicative value, $k1$ is the trunk upright indicative value, $k2$ is the leg upright indicative value, $k3$ is the supporting leg load reduction indicative value, $k4$ is the comfort indicative value, and $k5$ is the redundancy indicative value When $y = 0$, the user's CCR is correct, and when $y = 1$, the user's CCR is incorrect.

The calculation method for the trunk upright indicative value $k1$ is as follows: when the trunk upright status value collected by the motion data collection component is 0, $k1 = 0$, and when the trunk upright status value is 1, $k1 = 1$. The calculation method for the leg upright indicative value $k2$ is: when the leg upright status value collected by the CCR data collection component is 0, $k2 = 0$, and when the leg upright status value is 1, $k2 = 1$. The calculation method for the supporting leg load reduction indicative value $k3$ is as follows: determine whether the ratio of the supporting leg load reduction while on support of two sides of the body to body weight, *S* for short, or the ratio of the supporting leg load reduction while on support of the chest front to body weight, *N* for short, is within the range of the supporting leg load reduction in the given graduated treatment models, Δ*a* for short. If it is within the range, the output is $k3=0$; otherwise, the output is $k3=1$. The calculation method for the comfort indicative value is $k4 = round(\alpha Q + \beta S + \gamma T + \delta N)$, where *Q* is ratio of comfortable support point height on both sides of the body to body height, *S* is ratio of the supporting leg load reduction while on support of two sides of the body to body weight, *T* is ratio of comfortable support point height in front of the chest to body height, *N* is ratio of the supporting leg load reduction while on support of the chest front to body weight, and $\alpha, \beta, \gamma, \delta$ are model parameters obtained through parameter calibration, pre-set on the basis of user body data; and '$round(...)$' represents rounding. The redundancy indicative value $k5$ is an indicative value with a default value of 0.

The ratio of the supporting leg load reduction while on support of two sides of the body to body weight *S* refers to the ratio of reduced weight, collected when the user places his arms on both sides of his body and supports his body on the elbow, to the user's weight. The ratio of the supporting leg load reduction while on support of the chest front to body weight *N* refers to the ratio of the reduced weight, collected when the user places his arms in front of his chest and supports his body on the elbow. Different treatment models have different requirements for the supporting leg load reduction Δ*a*. Adjusting the height elbow supports or the user's support posture can keep the values of *S* and *N* within the range of Δ*a* assigned to a particular model. Weight data is collected through weight sensors mounted on the trunk support component.

The comfortable support point height ratio on both sides of the body to the height *Q* refers to the ratio of the elbow support point height, when the user places his arms on both sides of his body and supports his body on the elbow, to the user's height. The comfortable support point height ratio in front of the chest to the height *T* refers to the ratio of the elbow support point height, when the user places his arms in front of his chest and supports his body on the elbow, to the user's height. User height can be obtained through input or collected by sensors. The height of elbow supports can be adjusted manually by the user or automatically by a motor.

The comfort indicative value can be calculated based on the user's input of personal information or adult body inertia parameters. The ratio of the elbow support point height to shoulder height is between 0.79 and 0.91; the ratio of the elbow support point height to body height is between 0.65 and 0.75; The vertical plane distance between the comfortable stepping point and the elbow support point is between 29-45cm, concentrated around 35cm. The ratio of the vertical plane distance between the stepping point and the elbow support point to the length of the upper arm is between 0.9 and 1.4, mostly between 1.1 and 1.3. The value of the comfortable support point height ratio *Q* on both sides to the height is between 0.62 and 0.67, and the value of the comfortable support point height ratio *T* in front of the chest to the height is between 0.64 and 0.70. It is basically consistent with the analysis data of the 'dynamic positions during leg swinging' (the ratio of the elbow support point height to body height is 0.65-0.75). Whether in static support positions or during dynamic leg swinging, all test subjects report that supporting on both sides is more comfortable than supporting in front of the chest and it provides better leverage. The weight measurements in static support positions are basically within the range of the weight measurements during dynamic leg swinging. The ratio of maximum weight reduction while on support of both sides to body weight is between 0.10 and 0.66, and the ratio of minimum weight reduction while on support of both sides to body weight is between 0.08 and 0.36. The ratio of maximum weight reduction while on support of the chest front to body weight is between 0.03 and 0.49, and the ratio of maximum weight reduction while on support of the chest front to body weight is between 0 and 0.22. The values of the model parameters $\alpha, \beta, \gamma, \delta$ are calibrated and adjusted based on the values of *S, N,*

*Q, T* to ensure that *k*4=0 under the condition that the user feels comfortable and the limits of supporting leg load reduction is met; *k*4=1 when the user feels uncomfortable or the limits of supporting leg load reduction is not met. The formula used for calculating the comfort indicative value in this invention embodiment is *k*4=*round*(0.25*Q*+0.28*S*+0.15*T*+0.18*N*).

The treatment process control module matches the movement quantity, movement frequency and movement amplitude collected by the motion quantity data collection component with the corresponding values in the user's treatment model, and calls the corresponding feedback instructions in the feedback information storage module based on the matching results. When the user's CCR data conforms to the CCR analysis model, it records the movement as an effective one and triggers the corresponding feedback information. When the movement quantity conforms to the user's treatment model, it triggers the corresponding feedback information. When the quantity data of effective movements conforms to the user's treatment model, it triggers the corresponding feedback information, guiding the user to complete the daily treatment and rehabilitation exercise as prescribed by the treatment model. After the user completes a stage of treatment and rehabilitation exercise, the treatment process control module imports the next level of graduated treatment model. The feedback instructions include count standard feedback information, CCR standard feedback information, incentive feedback information, separation alarm information, and media information. The separation alarm information includes human-machine separation alarms and components-separated alarms. The human-machine separation alarm is used to remind the user to exercise as scheduled, by sounding an alarm at a preset time or/and voicing alarm information in accordance with the length of time the user being out of exercise. The components-separated alarm alarms when different components are placed too far apart to prevent loss of components. Multi media information is developed to help relieve restlessness of the user, including but is not limited to music, videos, online messages, video calls and live chat rooms.

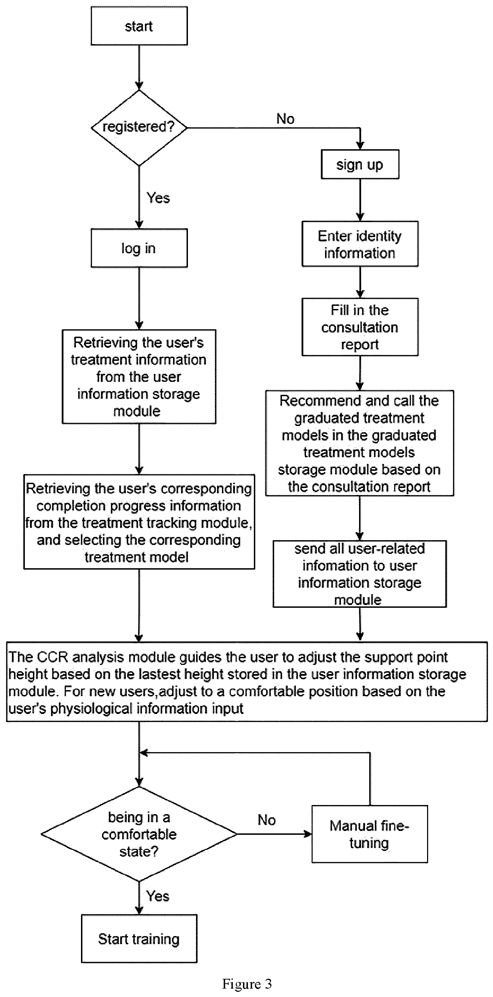

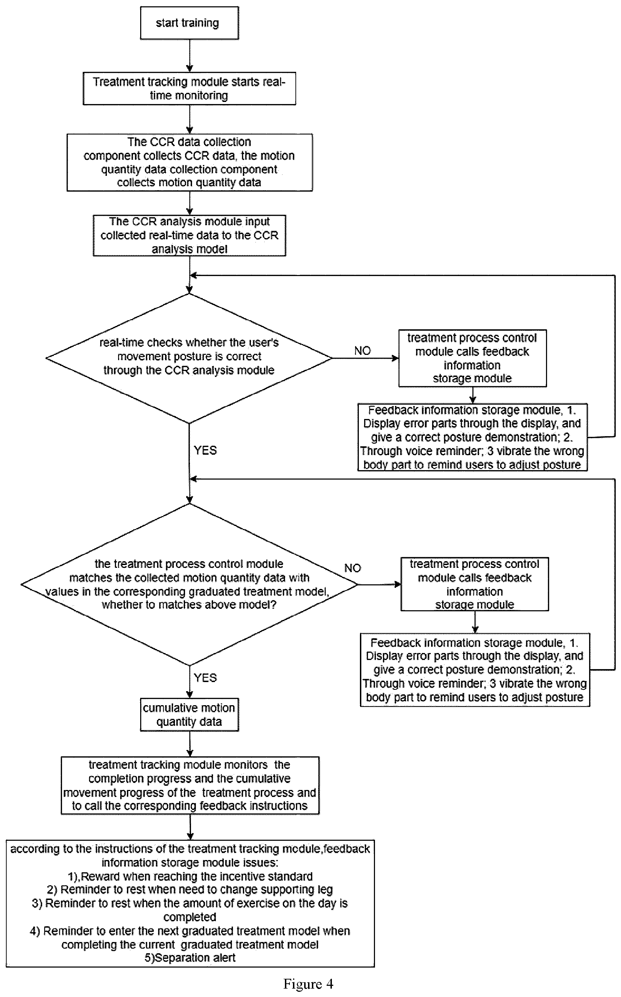

The workflow of the treatment process control module is shown in Figures 3 and 4, among which, the pre-exercise steps include:

1) Verifying the user's identity information, guiding him to log in to an account or to create an account, calling the user's treatment information from the user information storage module and the user's corresponding completion progress information from the treatment tracking module, and selecting the corresponding treatment model for degenerative knee joint disease from the treatment model storage module;

2) The CCR analysis module guides the user to adjust the elbow support point height and the lower leg length adjustment frame in the support component based on the comfortable support point height and data of weight reduction (as a result of being on support) stored in the user information storage module. For new users, the elbow support point height and the lower leg length adjustment frame in the support component are adjusted to a comfortable position based on the user's physiological information input.

3) Guide the user to start training in a comfortable state.

The steps of the training phase include:

1) The CCR data collection component collects CCR data, the motion quantity data collection component collects motion quantity data, and all data so collected are inputted to the CCR analysis model. The CCR analysis model calculates the user's movement posture based on the CCR data. When the CCR indicative value is 1, it outputs the corresponding feedback command, displays on the monitor corresponding positions and pictures of correct postures. If the upright posture indicative value *k*1 is 1, it outputs the voice message 'Please stand straight'; if the straight leg indicative value *k*2 is 1, it outputs the voice message 'Your leg is not straight, please stretch your leg straight and swing it'; if the supporting leg load reduction indicative value is 1, it outputs the voice message 'The pressure on your leg is too high, please adjust the elbow support to reduce the weight'; if the comfort indicative value *k*4 is 1, it outputs the voice message 'Are you feeling uncomfortable? Please adjust your posture.' The device prompts the user to make corrections to his posture, among other methods, by vibrating at incorrectly placed body parts.

(2) Real-time collect and record user motion quantity data and/or motion posture data, calling feedback instructions from the feedback information storage module to guide the user to complete standardized exercises. When the times of supporting the body on one leg *V* meets the standard, it outputs the voice message 'Please switch legs' or 'The supporting leg needs to rest'; When the rest time is shorter than the necessary supporting leg rest time *t,* it outputs the voice message 'The supporting leg needs to rest, please wait'; if the movement amplitude $\varphi$ does not meet the standard, it outputs the voice message 'The leg swing amplitude is not enough'; when the movement quantity of leg swinging cycles reaches a hundred, it outputs feedback information of encouragement, in forms of voice messages and sole massage.

(3) The motion process control module compares motion quantity data collected from the user with corresponding prescriptions of the user-specific treatment model until the former falls within the range of the latter.

(4) Store user motion quantity data and/or motion posture data, update the user's treatment information and the corresponding user's completion progress information in the treatment tracking module, and call the feedback instructions from the feedback information storage module to guide the user to end the process.

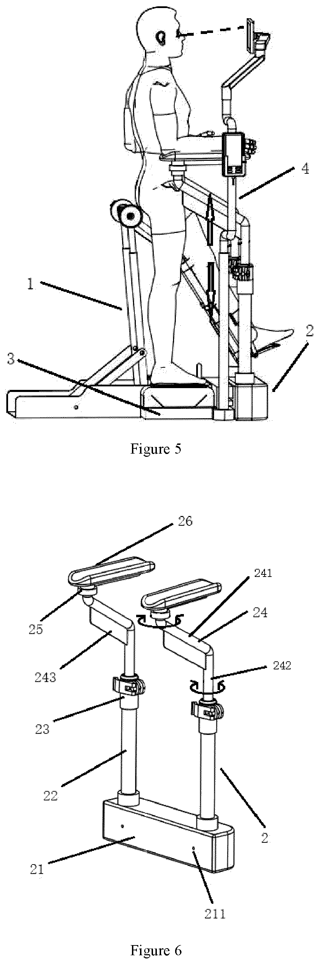

The support component includes: folding leg swing mechanism 1, multi-functional support adjuster 2, a multi-functional support adjuster 3, a terminal support frame 4, and a display 5. The CCR data collection component and the motion quantity data collection component are installed on the support component, and the control component is integrated into the display 5, as shown in Figures 5, 6, 7, 8, 9, 10, 11, 12, 13, 16, 17, 18. The multi-functional support frame 2 is used to support the user's elbows and is fixedly connected to the folding leg swing mechanism 1.

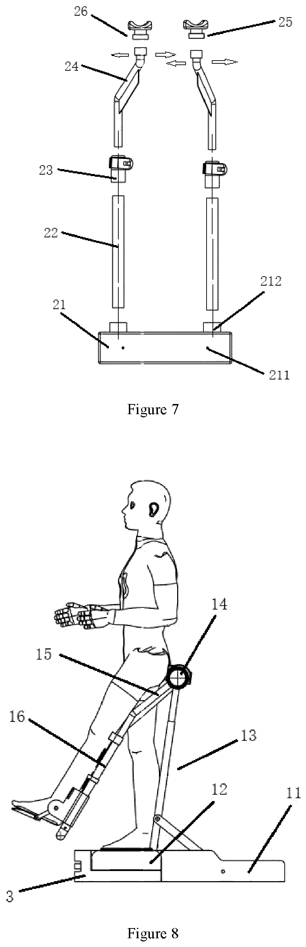

As shown in Figures 6 and 7, the multi-functional support frame 2 includes a support base 21, two support rods 22 and two elbow and armpit dual-use support bracket 26 which are paired. The support base 21 has guide rail mounting holes 211 for installing the multi-functional support adjuster 3 and support rod fixing holes 212 for connecting the support rods 22. One end of each of the two support rods 22 is fixedly connected to the support base 21 through the support rod fixing holes 212 and, relative to the center-line of the support base 21, the rods are symmetrically arranged. The elbow and armpit dual-use support brackets 26 are pivotally connected to the other ends of the rods 22, and the pivot axis of the brackets 26 are perpendicular to the pedal surfaces 121. As an example of embodying the invention, while the user is performing leg swing exercise in a standing position, he can rest his elbows or armpits on the two elbow and armpit dual-use support brackets 26 to ensure body balance and reduce the weight borne by the standing leg. One end of each of the elbow and armpit dual-use support brackets 26 is pivotally connected to one end of the support rods 22; and by rotating the elbow and armpit dual-use support brackets 26, the space between the elbow and armpit dual-use support brackets 26 can be adjusted to accommodate different user shoulder widths, which enhances further user comfort.

The treatment and rehabilitation device also includes two cam locking rings 23 and two support adjustment rods 24 each of which matches with one of the elbow and armpit dual-use support brackets 26. Each of the support adjustment rods 24 includes a third rod body 241 and a fourth rod body 242. One end of the third rod body 241 is pivotally connected to the matching elbow and armpit dual-use support bracket 26, while the other end of the third rod body 241 is connected to one end of the fourth rod body 242. The other end of the fourth rod body 242 is pivotally connected to the other end of the support rod 22 through the cam locking ring 23, and the pivot axis of the fourth rod body 242 is perpendicular to the pedal surface 121. The third rod body 241 and the fourth rod body 242 are arranged at a right angle or obtuse angle, with the second reinforcing flange 243 set between them to enhance stability. As an example of embodying the invention, a support adjustment rod 24 in the form of a folded rod is placed between the elbow and armpit dual-use support bracket 26 and the support rod 22. The elbow and armpit dual-use support bracket 26 is pivotally connected and locked to the third rod body 241 through the support bracket locking ring 25. While adjusting the rotation of the elbow and armpit dual-use support bracket 26 relative to the third rod body 241, the rotation of the fourth rod body 242 relative to the support rod 22 can also be further adjusted, and the fourth rod body 242 can ultimately be locked in place by the cam locking ring 23. This further expands the adjustment range of the elbow and armpit dual-use support bracket 26, making it more possible to accommodate different needs and deliver comfort.

As an example of embodying the invention, by adjusting the depth at which the fourth rod body 242 is inserted into the support rod 22, the overall height of the elbow and armpit dual-use support bracket 26 can be adjusted accordingly, making it more possible to accommodate users of different heights.

As shown in Figure 8, the folding leg swing mechanism 1 includes a housing 11, a foot pedal 12, a folding telescopic frame 13, a motor 14, a swing angle frame 15, and a lower leg length adjustment frame 16. The foot pedal 12 is mounted on the housing 11 and has a pedal surface 121 for the user to stand on. The folding telescopic frame 13 and the swing angle frame 15 are both rod-shaped. One end of the folding telescopic frame 13 is connected to the housing 11, and the other end of the folding telescopic frame 13 is hinged to one end of the swing angle frame 15 via a pivot 1a, which is parallel to the pedal surface 121. The motor 14 is mounted at the other end of the folding telescopic frame 13 and connected to the pivot 1a to provide it with driving force. The motor 14 is electrically connected to the control component. The other end of the swing angle frame 15 is connected to the lower leg length adjustment frame 16, which has a footrest plate 165a on it for supporting the foot.

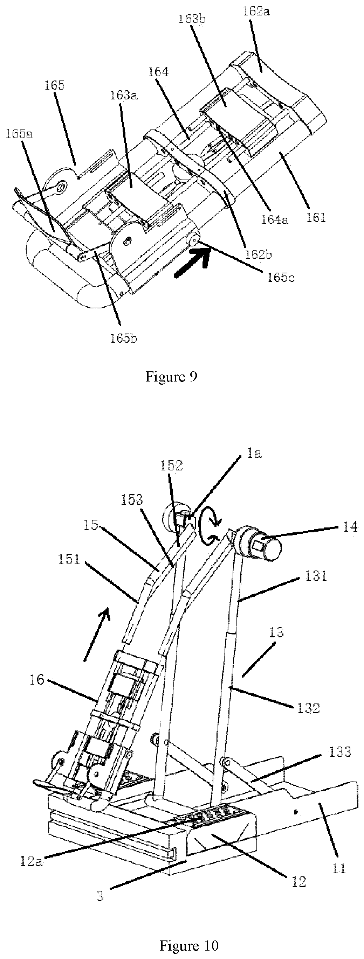

As shown in Figure 10, the swing angle frame 15 includes a first rod body 151 and a second rod body 152. One end of the first rod body 151 is connected to the lower leg length adjustment frame 16, and the other end of the first rod body 151 is connected to one end of the second rod body 152. The other end of the second rod body 152 is hinged to the other end of the folding telescopic frame 13, and the first rod body 151 and the second rod body 152 are arranged at an obtuse angle. As an example of embodying the invention, the swing angle frame 15 consists of the first rod body 151 and the second rod body 152 arranged in an obtuse included angle. As an example of embodying the invention, the obtuse included angle range is from 150° to 160°, and first reinforcing flange 153 is provided between the included angles to enhance stability. Since the thigh and the lower leg can not remain completely straight at all times, arranging the first rod body 151 and the second rod body 152 at an obtuse angle can make it better fit the human body's configuration, reduce interference from the swing angle frame 15 and the inside and outside of the thigh, and render the hip joint as close as possible to the pivot 1a during exercise, thereby improving the comfort standard of the embodiment.

As an example of embodying the invention, the folding leg swing mechanism 1 includes two folding telescopic frames 13, two swing angle frames 15. Two sets of swing mechanisms, each of which is composed of a swing angle frame 15 and lower leg length adjustment frame 16, jointly drive the lower leg length adjustment frames 16 into pendulum motion. This design improves the structural stability of the embodiment.

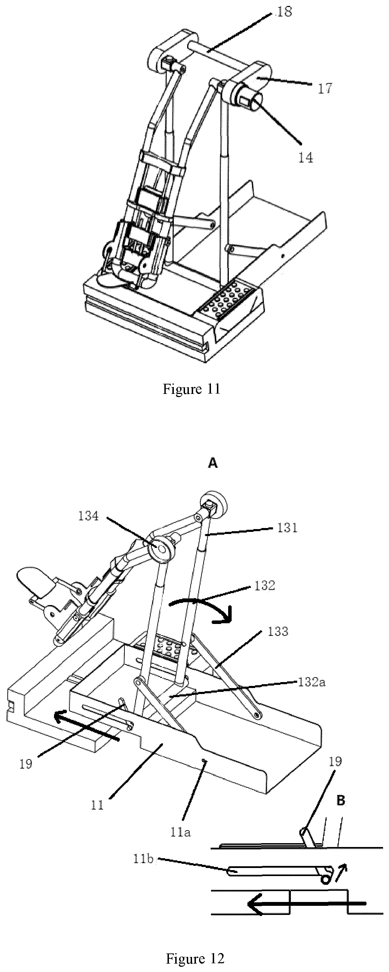

In this exemplary embodiment of the present invention, there is a problem of poor stability when using the motor 14 to drive the corresponding swing angle frame 15. As shown in Figure 11, it is viable to add two gear cases 17 respectively at the places where the other end of the two folding telescopic frames 13 are hinged to the swing angle frames 15, and connect the power of the two gear cases 17 through the coupling 18. The coupling 18 can be installed in a sleeve between the two gear cases 17 for better concealment and protection. In this configuration, only one motor 14 is needed to drive the pivot 1a at the hinge where the swing angle frames 15 meet their counterpart folding telescopic frames 13. The motor 14 transfers power to the gear case 17 on one side and then, through the coupling 18, the power is transmitted to the swing angle frame 15 on the other side. With the power on both sides being thus synchronized, this embodiment works two-drive on both left and right sides with enhanced in-motion stability of the swing angle frame 15. When the coupling 18 moves backward, the pivot center of the swing angle frame 15 remains stationary. This consequently brings the pivot center of the swing angle frame 15 further closer to the hip joint, makes the trajectory of the swing angle frame 15 more consistent with the that of leg swinging, improving the comfort standard experienced by the user in the process of the rehabilitative exercise and ensuring smooth forward and backward swinging motion of the legs.

As shown in Figure 9, the lower leg length adjustment frame 16 includes a U-shaped frame 161, a guide rod 164, and a first slider 165. One end of the U-shaped frame 161 is connected to the first rod body 151 in a detachable manner. The guide rod 164 is set on the U-shaped frame 161 and parallel to the first rod body 151. The first slider 165 has a first guide hole 164a, and it can be mounted on the U-shaped frame 161 in a slide and connected to the guide rod 164 through the first guide hole 164a.

The footrest plate 165a is mounted on the first slider 165, and the first slider 165 also has a locking mechanism 165c for connection in a fixed way with the U-shaped frame 161. Illustratively, as shown in Figure 10, in this embodiment of the present invention, the lower leg length adjustment frame 16 is connected to the two swing angle frames 15's first rod body 151 through the two ends of the U-shaped frame 161. The footrest plate 165a installed on the first slider 165 can be slid along the length of the guide rod 164 on the U-shaped frame 161 to adjust its relative position with the U-shaped frame 161, to accommodate the leg length of users of different heights. After adjusting to the user's comfortable foot position, the first slider 165 is fixedly connected to the U-shaped frame 161 through the locking mechanism 165c, completing the adjustment and fixation of the footrest plate 165a position, further enhancing the user's comfort during use.

Illustratively, in this embodiment of the present invention, the lower leg length adjustment frame 16 also includes a first securing frame 162a, a third slider 163b, a second securing frame 162b, and a second slider 163a. The first securing frame 162a, the third slider 163b, the second securing frame 162b, the second slider 163a, and the first slider 165 are arranged along the length of the guide rod 164 and are all provided with guide holes for connecting the guide rod 164. The first securing frame 162a and the second securing frame 162b are fixedly connected to the U-shaped frame 161, and the positions of the third slider 163b and the second slider 163a can be adjusted by sliding relative to the U-shaped frame 161 along the length of the guide rod 164, in order to provide support for the user's calf and to do this with enhanced comfort.

In Figure 9, the folding leg swing mechanism 1 is in its longest state, and the first slider 165 can slide in the direction indicated by the arrow to adjust and accommodate the length of the lower leg. When the first slider 165 reaches the second slider 163a, the second slider 163a and the third slider 163b move in opposite directions under the action of the linkage. The sliders and securing frames serve as support for the calf. The first slider 165 has a locking mechanism 165c; and when the first slider 165 slides on the U-shaped frame 161 to the position where the user feels comfortable, it can be locked in place by the locking mechanism 165c.

Illustratively, in this embodiment of the present invention, a plate linkage 165b is used to connect the footrest plate 165a with the first slider 165, as shown in Figures 7, 8, and 9. One end of the plate linkage 165b is connected to the footrest plate 165a through a torsion spring, and the other end of the plate linkage 165b is connected to the first slider 165 through a torsion spring. The rotation shafts at both ends of the support plate link 165b are parallel to the pivot 1a. The footrest plate 165a expands when subjected to pressure from the foot. By applying the above-mentioned dual adjustable pivot design for the footrest plate 165a and the first slider 165, on account of the fact that the rotational centers of the human hip and the swing angle frame15 cannot be completely coaxial, there is deviation in the trajectories of the foot swinging during exercise and the lower leg support frame. The footrest plate 165a installed in this configuration can rotate in real time according to the trajectory of the foot during swinging, avoiding effects of pressing and pulling on the foot due to out-of-sync trajectories. After exercise, the footrest plate 165a can be reset under the action of the torsion spring, which further raises the user's comfort standard.

Illustratively, in this embodiment of the present invention, as shown in Figures 10 and 12, the folding telescopic frame 13 includes a telescopic rod 131, a folding frame 132, and an housing linkage 133. As can be seen from the perspective view A in Figure 12, one end of the telescopic rod 131 is hinged to the Swing angle frame 15 through the pivot 1a, and the other end of the telescopic rod 131 can be telescopically inserted into the folding frame 132. The housing 11 comes with pivot holes 11a on its body which are used to connect the housing linkage 133; and the axes of pivot holes 11a are parallel to the pivots 1a. One end of each of the housing linkages 133 is connected to the corresponding pivot hole 11a through rotation shafts, and the other end of each of the housing linkages 133 is hinged to the middle of the corresponding folding frame 132, with the hinge axes of the other end of the housing linkages 133 being arranged respectively parallel to their corresponding pivot 1a. The housing 11 also has housing linkage-fastening holes 11b parallel to the pedal surfaces 121, and one end of the folding frames 132 is to be mounted by a slide into the housing linkage-fastening holes 11b through the linkage pivot 132a. Seeing in combination of the perspectives views A and B, it is known that two housing linkage-fastening holes 11b are symmetrically arranged on both sides of the housing, and the axes of the folding frames 132 pass through the length and in the middle of the frame bodies and lead to the housing linkage-fastening holes 11b on the upper sides of the housing. By sliding the linkage pivot 132a of the folding frame 132 in the housing linkage-fastening holes 11b, the folding telescopic frame 13 can be folded and stored in the housing 11, as shown in perspective view B of Figure 12, and locked in place by the locking handle 19 on the linkage pivot 132a, thus the folding leg swing mechanism 1 is folded for convenient storage and easy carrying.

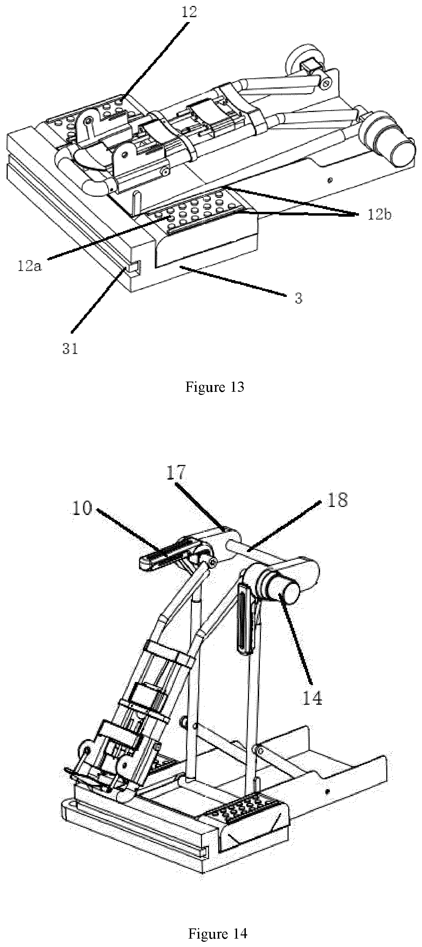

Illustratively, in this embodiment of the present invention, Figure 13 shows the folded state of the folding leg swing mechanism 1. On the foot pedal surfaces 121 of the foot pedals 12 are massage protrusions 12a being arranged in even intervals; and on both sides of the foot pedal surfaces 121, anti-slip protrusions 12b are arranged; these protrusions provide both massaging and anti-slip effects for the user when he/she steps on the foot pedal 12, keeping the user from slipping and improving the safety and comfort standard of this therapeutic and rehabilitative device.

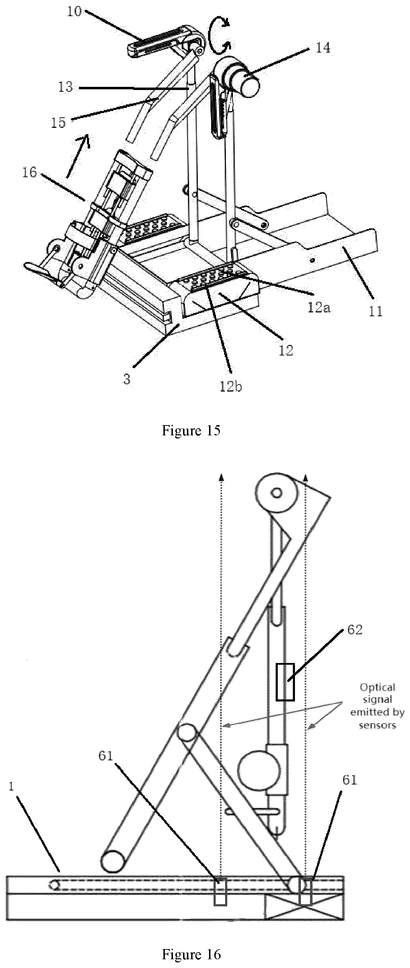

As shown in Figure 14, this therapeutic and rehabilitative device also includes a flip-folding seat 10, which is pivotally connected to the other end of the folding and telescopic frame 13; and the pivot axis of the flip-folding seat 10 is coaxial with the pivot 1a; and the flip-folding seat 10 is used to support the user's buttocks. In another example of possible implementations, as shown in Figure 15, a flip-folding seat 10 which is pivotally connected is set at the other end of the folding and telescopic frame 13. For users with difficulty to stand, the flip-folding seat 10 can be rotated around the pivot 1a to a horizontal position to provide auxiliary support for the user's buttocks during exercise, allowing the user to perform the leg swinging movements in a seated position and thus further improving the practicality of this therapeutic and rehabilitative device.

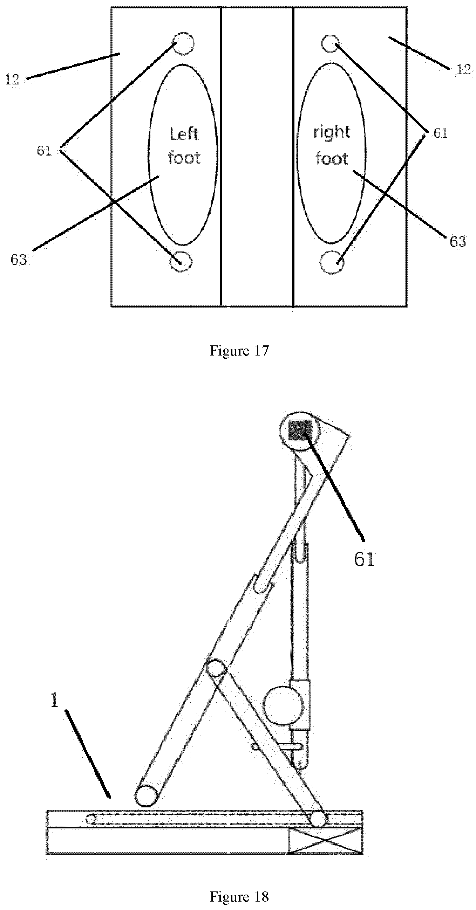

Illustratively, in the present embodiment of the invention, the CCR data collection component includes the first sensor group 61, the second sensor group 62, and the third sensor group 63, used to collect the CCR data of the user in use, and the motion quantity data collection component includes the fourth sensor group 64 used to collect the user's motion data in use. The CCR data collection component and the motion data collection component are both electrically connected to the control component. The first sensor group 61 includes photoelectric sensors set in front and behind the foot pedals 12 of the support component, as shown in Figure 16, with the receiving end of the photoelectric sensor vertically positioned above the two transmitting ends. When the user performs leg swinging exercise, if the receiving end receives the photoelectric signal which is not blocked by the user, the output for trunk upright status value is 0, $k1=0$. When the receiving end does not receive the photoelectric signal due to it being blocked by the user, the output for the upright trunk status value is 1, $k1=1$. The two sets of photoelectric sensors are respectively assigned to eye the left and right feet, and the distance between them can be adjusted according to practical requirements. The sensors transmit photoelectric signals upwards in vertical direction. When the user is in the position of standing upright, When a person is in an upright position, the transmitted signals will not be blocked by the body. When the user is in a bent position, the signals transmitted by the sensors will be blocked by the body; and the sensor can detect the signals reflected back, thereby determine the presence of an obstacle and then determine that the body is not upright; in this case, it outputs $k1=1$. If no reflected signal is detected, it determines that the body is in an upright state; in this case, it outputs $k1=0$. There are four sensors for observing leg swinging in an upright position, and the installation diagram is shown in the top-view Figure 17. When a person stands on the left foot and swings the right, if the sensor on the left side does not detect a reflected signal, it indicates that the left leg is upright. If the sensor on the right side detects reflected signals that are periodically changing, it indicates that the right leg is swinging. If the sensor on the right side does not detect any reflected signal, it indicates that the right leg is in an upright position. It is important to note that the beam angle of the sensor signal is very narrow. If a person is in a bent or hunched position, the transmitted signal can be reflected back and detected by the sensor, thus it can be determined that the person is not in an upright position.

The first sensor group 61 can also be implemented by setting up a TOF sensor at the top pivot 1a of the folding telescopic frame 13 on the folding leg swing mechanism 1, which shoots searching signals towards the upper part of the human body, as shown in Figure 18. It shoots searching signals towards the upper body of the human (as shown in the figure) and collects depth signals of this part of the space (the line formed by the closest distance to the TOF sensor). When the TOF sensor collects a depth signal perpendicular to the ground, the body is in an upright position, and the output for the upright trunk status is 0, $k1=0$. If the collected depth signal is not perpendicular to the ground, it indicates that the upper body is in a bent position and not upright, and the output for the upright trunk status is 1, $k1=1$. The TOF sensor is mainly responsible for detecting whether the upper body is upright. Whether the thighs and calves are upright can still be detected by the four photoelectric sensors installed at the foot pedal position. The photoelectric sensors detect whether there are obstructions below the thighs. If the photoelectric sensors detect an obstruction, it indicates that the thighs and calves are in a bent position and not upright.

The second sensor group 62 includes displacement sensors placed on the lower leg length adjustment frame 16 corresponding to the back of the user's knees, as shown in Figure 16. The displacement sensors continuously collect the fitting status of the user's legs with the lower leg length adjustment frame16. When the legs are fitted to the frame, the output for the upright status of the legs is 0, $k2=0$. When they are not, the output for the upright status of the legs is 1, $k2=1$.

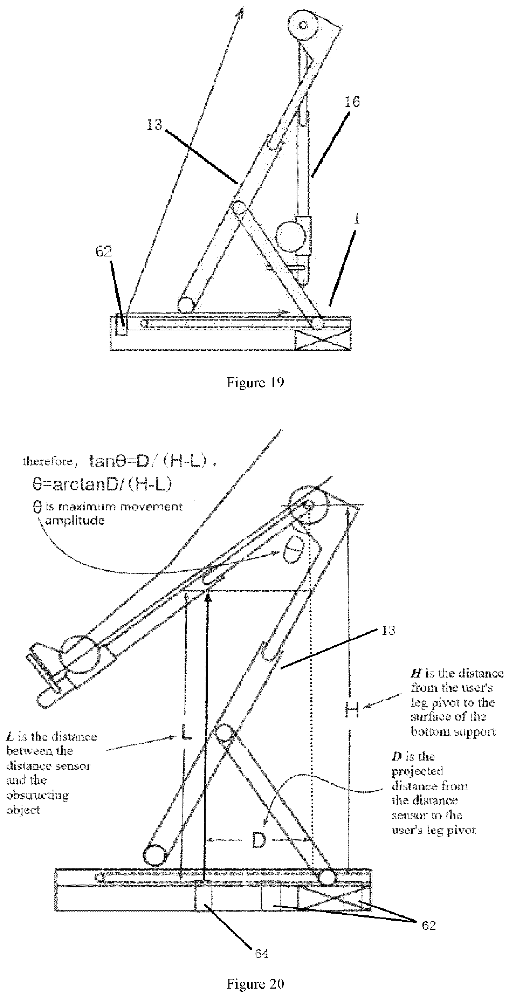

Through a camera, the second sensor group 62 can be implemented as follows: as shown in Figure 19, the camera is installed at the front of the support component to recognize the user's posture and movements. The control system can recognize the user's posture and movements by the 'Openpose' system. When the user is performing leg swing exercises, the camera can identify whether the exerciser is doing upright leg swings, and output the upright trunk status value and leg upright status value.

The third sensor group 63 includes weight sensors set below the foot pedals of the support component, as shown in Figure 18, to real-time collect the user's weight data. The method for calculating the supporting leg load reduction indicative value is based on whether the ratio of the supporting leg load reduction while on support of two sides of the body to body weight, $S$ for short, or the ratio of the supporting leg load reduction while on support of the chest front to body weight, $N$ for short, is within the range of the supporting leg load reduction in the given graduated treatment models, $\Delta a$ for short. If it is within the range, the output is $k3=0$; otherwise, the output is $k3=1$.

The fourth sensor group 64 includes distance sensors, set at the front of the multifunctional support frame 2, and photoelectric sensors on the side of the housing 11 (which can be reused with the first sensor group 61). When measuring the movement amplitude of the leg swinging, as shown in Figure 20, the movement amplitude $\theta=arctan(D/(H-L))$, where $H$ is the distance from the user's leg pivot to the surface of the foot pedal, $L$ is the distance between the distance sensor and the obstructing object, and $D$ is the projected distance from the distance sensor to the user's leg pivot.

If you want the function of measuring swing amplitude, an angle sensor can also be installed at the pivot of the device. It can measure the swing range of the device and thereby the movement amplitude $\theta$ can be obtained. When measuring the number of leg swings made by the user, the photoelectric sensor records the number of movements and the movement frequency by detecting the periodic changes in the signals.

The control component is configured to control the motor 14 according to the type of leg assistance $F$ prescribed in the particular graduated treatment models, driving the swing angle frame 15 to rotate. When the leg assistance force $F$ in the graduated treatment models is a complementary driving force, the motor 14 drives the swing angle frame 15 to rotate. When the leg assistance $F$ is the primary driving force, the motor 14 does not provide power to the swing angle frame 15 and it is the user's leg which drives the swing angle frame 15 to rotate. When the leg assistance $F$ is a resistance force, the motor 14 provides reverse power to the swing angle frame 15, and the user overcomes the resistance by working the leg and thereby drives the swing angle frame 15 to rotate.

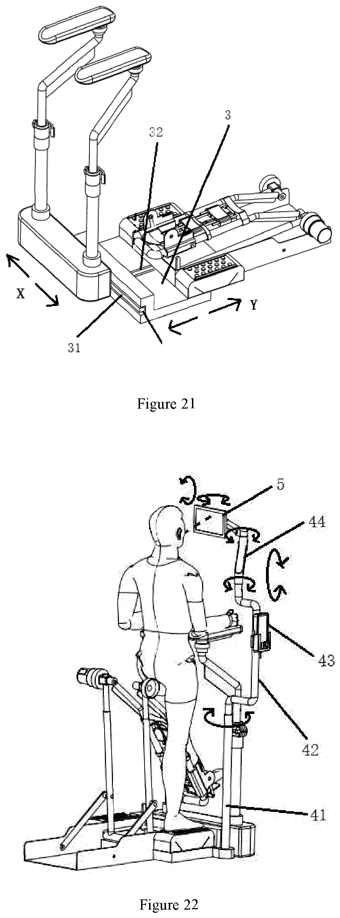

As shown in Figure 21, this therapeutic and rehabilitative device also includes a multifunctional support adjuster 3 which is connected to the housing 11. The multifunctional support adjuster 3 has a first guide rail 31 parallel to the pivot 1a, and the support base 21 can be connected to the first guide rail 31 through a sliding groove. The folding leg swing mechanism 1 includes two foot pedals 12 symmetrically arranged with respect to the lower leg length adjustment frame 16. Illustratively, in this embodiment of the present invention, by setting a foot pedal 12 on each side of the folding leg swing mechanism 1, the user can, based on his/her own needs, either stand on the left leg on one foot pedal 12 and work the right leg to do swinging exercise on support of the lower leg length adjustment frame16, or stand on the right leg on the other foot pedal 12 and use work the left leg to do swinging exercise on support of the lower leg length adjustment frame16. During swinging exercise with the right leg, the position of the support base 21 can be adjusted along the length of the first guide rail 31 to move the multifunctional support frame 2 into positions to the left of the multifunctional support adjuster 3 and fixedly connected to it. During swinging exercise with the left leg, the position of the support base 21 can be adjusted along the length of the first guide rail 31 to move the multifunctional support frame 2 into positions to the right of the multifunctional support adjuster 3 and fixedly connected to it. This ensures that the two elbow and armpit dual-use support brackets 26 on the multifunctional support frame 2 can be moved into a position convenient for the user to support his/her elbows and armpits, therefore improves the practicality and user comfort standard of the therapeutic and rehabilitation device.

Illustratively, in this embodiment of the present invention, the multifunctional support adjuster 3 can also be fitted with a second guide rail 32 perpendicular to the first guide rail 31, and the housing 11 can be installed on the second guide rail 32 through a sliding groove. To suit his/her own needs, the user can adjust the relative position of the housing 11 in the length direction of the second guide rail 32, thus alter the distance between the folding leg swing mechanism 1 and the multifunctional support frame 2. This helps achieve tailored adjustments of the standing space to accommodate users of different body types, and further enhances the practicality and user comfort standard of the therapeutic and rehabilitation device.

As shown in Figure 21, during rehabilitative exercise of the left leg on the leg swing mechanism, the user's right foot steps on the right foot pedal. When the user exercises with the right leg, the left foot steps on the left foot pedal; the user has to move half a body's position to the left. Under the circumstance, the position of the multifunctional support frame 2 needs to be adjusted simultaneously to be able to provide support along the correct force line. The horizontal movement function in the X direction can solve this problem. Joint adjustments in the X and Y directions not only changes the relative support position, but also increases or decreases the user's standing space when moving in the Y direction. Therefore, the multifunctional support adjuster 3 can meet the needs of users of different heights and body types, greatly improving the device 's usability.

As shown in Figure 22, the terminal support frame 4 includes a straight frame 41, a rotating U-shaped frame 42, and an all-direction adjustment bracket 44. The straight frame 41 is perpendicular to the foot pedal surface 121 and is fixedly connected to the multifunctional support adjuster 3 at one end. The other end of the straight frame 41 is connected to the rotating U-shaped frame 42, and the other end of the rotating U-shaped frame 42 is connected to the all-direction adjustment bracket 44. A remote control holder 43 is provided on the rotating U-shaped frame 42 for placing the device's remote control, and the other end of the all-direction adjustment bracket 44 is used to support the display 5.

When the user needs to use this therapeutic and rehabilitation device for rehabilitative exercise, he/she can stand in a single-leg stance on the foot pedal surface 121 of the folding leg swing mechanism 1, fasten the leg that needs to be exercised to the swinging structure composed of the swinging angle frame 15 and the lower leg length adjustment frame 16. The foot of the latter can step on the footrest plate 165a for support and positioning. While standing, the user can rest his/her elbows or armpits on the multifunctional support frame 2 to maintain balance and lighten the weight burden on the standing leg. Then, the user can perform the rehabilitative exercise either by, through his/her own strength, making the lower leg length adjustment frame 16 and swing angle frame 15 revolve together around pivot 1a in pendulum motion relative to the folding telescopic frame 13, or with the help of the device which controls the motor 14 to, through a control component and according to a pre-set program, drive the lower leg length adjustment frame 16 and swinging angle frame 15 into pendulum motion. Meanwhile, the first sensor group 61, the second sensor group 62, and the third sensor group 63 on the foot pedal 12 collect the user's CCR data, which includes the comfortable support height, supporting leg load reduction, trunk upright status value and upright leg status value. The fourth sensor component 64 on the housing 11 collects the user's motion data, including the movement quantity of leg swings, the frequency and amplitude of motion, and send the CCR and motion quantity data to the control component for purposes of collection and analysis. The treatment process control module in the control component will call the corresponding feedback instructions in the feedback information storage module based on the CCR judgment instructions; It matches the movement quantity, movement frequency, and movement amplitude collected by the motion quantity data collection component with the corresponding values in the treatment model, and calls the corresponding feedback instructions in the feedback information storage module based on the matching results, with a view to controlling the motor 14 to move in a certain speed and frequency. Thus, the swinging speed and amplitude of the leg are ensured to be within the appropriate range, thereby improving therapeutic and rehabilitative effects.

When the control component receives the CCR data collected by the first sensor group 61, it can also input the real-time collected CCR data into the CCR analysis model, to real-time determine the user's posture information, and to output CCR judgment instructions; When the user's CCR is found to be unsatisfactory, it simultaneously outputs an incorrectness flag.

Finally, it prompts the user through voice, video, or tactile messages to adjust the body posture up to standard in order to ensure effectiveness of the rehabilitative exercise.

The above-mentioned rehabilitative device is a decent example of embodiment of this invention. Notwithstanding, the embodiment of the invention is not subject to the above example. The scope of the invention is defined by the appended claims.

**Claims**

1.    A KOAPT-therapy-based treatment and rehabilitation device for degenerative knee joint disease, the device being designed based on knee osteoarthritis pendulum therapy, the therapy requiring a user to perform leg swinging movements about a user's leg pivot as a treatment and rehabilitation exercise, **characterized in that** the device comprises a Correct and Comfortable Relationship (CCR) data collection component, a motion quantity data collection component, a support component, a control component, and a feedback component; wherein:

the support component is used to provide trunk support and/or waist support and/or elbow support for the user; the CCR data collection component is installed on the support component to collect CCR body data of the user during therapy; the CCR refers to the relationship between correct and comfortable body postures subject to KOAPT requirements and body movement forms in motion status; the CCR body data comprises elbow support point height, supporting leg load reduction, trunk upright status value, and leg upright state value;

the motion quantity data collection component is installed on the support component to collect a motion quantity data of the user's leg swinging movements during use in real time; the motion quantity data comprises the number of leg swinging movements, movement frequency, and movement amplitude;

the control component is used to execute a KOAPT computer program installed in the control component, real-time collect the CCR body data of the user in use, and real-time determine whether the elbow support point height, supporting leg load reduction, trunk upright status value, and upright leg status value during the user's exercise match preset CCR values;

when the user's CCR body data doesn't match the KOAPT requirements, the feedback component receives feedback instructions from the control component and communicates corresponding information to the user, to correct the user's movement posture; the computer program also receives the motion quantity data sent by the motion quantity data collection component in real-time, and guides the user to complete the treatment and rehabilitation exercise according to graduated treatment models;

the feedback component is used to send audio and/or video and/or tactile signals to the user according to the feedback instructions from the control component;

the graduated treatment models consist of $X1$, $X2$ and $X3$ level treatment models; each graduated treatment model has preset multiple parameters, including: graduated period, single-leg support count $V$, movement amplitude $\theta$, movement frequency $\omega$, leg assistance force $F$, movement quantity $C$, necessary supporting leg rest time $t$, and supporting leg load reduction $\Delta a$;

the support component comprises a folding leg swing mechanism (1), including a housing serving as a bottom support, and a multifunctional support frame (2); the folding leg swing mechanism (1) is used to support the user's torso, while the multifunctional support frame (2) is used to support the user's elbows, and both are fixedly connected to each other;

the motion quantity data collection component is configured to collect the movement amplitude by means of a distance sensor placed on the upper surface of the bottom support, and a photoelectric sensor placed on a side of the housing for detecting an obstruction below the thigh of the user's leg;

the movement amplitude $\theta$ is calculated as $\theta=arctan(D/(H-L))$, wherein $H$ is the distance from the user's leg pivot to the upper surface of the bottom support, $L$ is the distance between the distance sensor and the obstruction, and $D$ is the projected distance from the distance sensor to the user's leg pivot.

2. The KOAPT-therapy-based treatment and rehabilitation device for degenerative knee joint disease of claim 1, **characterized in that** the KOAPT computer program comprises a graduated treatment models storage module, a CCR analysis module, a treatment process control module, a treatment tracking module, a feedback information storage module, and a user information storage module;

the graduated treatment models storage module is used to store the graduated treatment models;

the CCR analysis module inputs the real-time collected CCR body data into a CCR analysis model, real-time determines the user's posture information, and output CCR judgment instructions; when the user's CCR is incorrect, it simultaneously outputs an incorrectness flag;

the treatment process control module is used to call the corresponding feedback instructions in the feedback information storage module based on the CCR judgment instructions; the treatment process control module matches the number of leg swinging movements, movement frequency, and movement amplitude collected by the motion quantity data collection component with the corresponding values in the graduated treatment models, and calls the corresponding feedback instructions in the feedback information storage module based on the matching results;

the treatment tracking module monitors the progress and completion of the user's treatment process and to call the corresponding feedback instructions in the feedback information storage module;

the feedback information storage module comprises count standard feedback information, CCR standard feedback information, incentive feedback information, separation alarm information, and media information;

the user information storage module stores the user's personal information, CCR body data, and motion quantity data.

3. The KOAPT-therapy-based treatment and rehabilitation device for degenerative knee joint disease of claim 2,

**characterized in that** the CCR analysis model is represented by the

$$\text{equation } y = k1 + k2 + k3 + k4 + k5,$$

wherein $y$ is the user's CCR indicative value,
$k1$ is trunk upright indicative value,
$k2$ is leg upright indicative value,
$k3$ is supporting leg load reduction indicative value,
$k4$ is comfort indicative value, and
$k5$ is redundancy indicative value;
When $y = 0$, the user's CCR is correct, and when $y = 1$, the user's CCR is incorrect.

4.   The KOAPT-therapy-based treatment and rehabilitation device for degenerative knee joint disease of claim 3, **characterized in that** a calculation formula for the comfort indicative value is $k4 = round\ (\alpha Q + \beta S + \gamma T + \delta N)$,

wherein $Q$ is ratio of elbow support point height on both sides of the body to body height, $S$ is ratio of the supporting leg load reduction while on support of two sides of the body to body weight, $T$ is ratio of elbow support point height in front of the chest to body height, $N$ is the ratio of the supporting leg load reduction while on support of the chest front to body weight, and
$\alpha, \beta, \gamma, \delta$ are model parameters obtained through parameter calibration, pre-set on the basis of user body data; and '$round(...)$' represents rounding.

5.   The KOAPT-therapy-based treatment and rehabilitation device for degenerative knee joint disease of claim 1, **characterized in that** the folding leg swing mechanism (1) comprises a shell (11), two foot pedals (12), two folding telescopic frames (13), a motor (14), two swing angle frames (15), and a lower leg length adjustment frame (16);

the shell (11) serves as the bottom support, with the two foot pedals (12) mounted on the shell (11); the two folding telescopic frames (13) vertically set above the shell (11) are fixed by housing linkage (133); upper ends of the two swing angle frames (15) are respectively suspended on the folding telescopic frames (13) to achieve back and forth swinging relative to the folding telescopic frames (13); lower ends of the two swing angle frames (15) are inserted into the lower leg length adjustment frame (16) and fixed into a single unit; the motor (14) is located at the upper end of one of the swing angle frames (15), driving the swing angle frames (15) and lower leg length adjustment frame (16) to swing back and forth;
the control component is configured to control the motor (14) based on the graduated treatment models, thereby driving the swing angle frame (15) to rotate.

6.   The KOAPT-therapy-based treatment and rehabilitation device for degenerative knee joint disease of claim 1, **characterized in that** the multifunctional support frame (2) comprises a support base (21), two support rods (22), and two elbow and armpit dual-purpose support brackets (26); the support base (21) is located at a bottom of the multifunctional support frame (2), the two support rods (22) symmetrically positioned above the support base (21); the support rods (22) are connected to a support adjustment rod (24) via a cam handle, and a reinforcing flange is arranged below the support adjustment rod (24); the elbow and armpit dual-purpose support brackets (26) are horizontally positioned at a top of the support adjustment rod (24).

7.   The KOAPT-therapy-based treatment and rehabilitation device for degenerative knee joint disease of claim 5, **characterized in that** the lower leg length adjustment frame (16) comprises a U-shaped frame (161), guide rods (164), and a first slider (165); the lower leg length adjustment frame (16) is connected to the two swing angle frames (15) through two ends of the U-shaped frame (161), respectively; a footrest plate (165a) mounted on the first slider (165), and slides along a length direction of the guide rod (164) on the U-shaped frame (161) to adjust its relative position to the U-shaped frame (161), in order to accommodate the leg length of users of different heights.

8.   The KOAPT-therapy-based treatment and rehabilitation device for degenerative knee joint disease of claim 1, **characterized in that** the device further comprises a terminal support frame (4) and a display screen, the terminal support frame is used to support and adjust height and angle of the display screen.

9.   The KOAPT-therapy-based treatment and rehabilitation device for degenerative knee joint disease of claim 5, **characterized in that** two flip-folding seats (10) are respectively installed on tops of the two folding telescopic frames (13), and the two flip-folding seats (10) are connected through a gear case (17) and a coupling (18).

10. The KOAPT-therapy-based treatment and rehabilitation device for degenerative knee joint disease of claim 5, **characterized in that** a flip-folding seat (10) is installed on a top of the folding telescopic frame (13), and the flip-folding seat (10) is hinged with an upper end of the folding telescopic frame (13).

11. The KOAPT-therapy-based treatment and rehabilitation device for degenerative knee joint disease of claim 1, **characterized in that** the CCR data collection component comprises a first sensor group (61), a second sensor group (62), and a third sensor group (63) for collecting body data of the user, and the motion quantity data collection component comprises a fourth sensor group (64) for collecting the user's motion quantity data.

12. The KOAPT-therapy-based treatment and rehabilitation device for degenerative knee joint disease of claim 11, **characterized in that** the first sensor group (61) comprises photoelectric sensors placed in front of and behind the foot pedals (12) on the support component; two receiving ends of the photoelectric sensors are vertically positioned above its two emitting ends respectively; when the user performs leg swinging movements, if the receiving end receives the photoelectric signal which is not blocked by the user, the first sensor group output a value of 0 for trunk upright status, i.e. $k1=0$; when the receiving end does not receive the photoelectric signal due to it being blocked by the user, the first sensor group output a value of 1 for the upright trunk status, i.e. $k1=1$.

13. The KOAPT-therapy-based treatment and rehabilitation device for degenerative knee joint disease of claim 11, **characterized in that** the second sensor group (62) comprises displacement sensors placed on the lower leg length adjustment frame corresponding to the back of the user's knees; the displacement sensors continuously collect a fitting status of the user's leg with the lower leg length adjustment frame; when the leg is fitted to the frame, the second sensor group output a value of 0 for the upright status of the leg, i.e. $k2=0$; when they are not, the second sensor group output a value of 1 for the upright status of the leg, i.e. $k2=1$.

14. The KOAPT-therapy-based treatment and rehabilitation device for degenerative knee joint disease of claims 3 11, **characterized in that** the third sensor group (63) comprises weight sensors placed under foot pedals (12) of the support component to continuously collect the user's weight data;
the CCR data collection component is configured to calculate the supporting leg load reduction indicative value k3 based on whether the ratio of the supporting leg load reduction while on support of two sides of the body to body weight, $S$ for short, or the ratio of the supporting leg load reduction while on support of the chest front to body weight, $N$ for short, is within the range of the supporting leg load reduction in the given graduated treatment models, $\Delta a$ for short; if it is within the range, the output is $k3=0$; otherwise, the output is $k3=1$.

15. The KOAPT-therapy-based treatment and rehabilitation device for degenerative knee joint disease of claim 11, **characterized in that** the fourth sensor group (64) comprises the distance sensor positioned at the front part of the multifunctional support frame (2) and the photoelectric sensor on a side of the housing (11).

16. The KOAPT-therapy-based treatment and rehabilitation device for degenerative knee joint disease of claim 6, **characterized in that**, when the comfort indicative value is determined to be 1, the control component commands the feedback component to issue audio and/or video and/or tactile signals to the user, guiding him/her in adjusting the height of a waist support and/or elbow support, or automatically adjusts the height of the waist support and/or elbow support until the comfort indicative value is determined to be 0.

17. The KOAPT-therapy-based treatment and rehabilitation device for degenerative knee joint disease of claim 1, **characterized in that** the CCR data collection component and the motion quantity collection component function through the integrated work of different parts, including ultrasonic sensor, displacement sensor, gravity sensor, photoelectric sensor, radar, and/or camera.

**Patentansprüche**

1. Auf KOAPT-Therapie basierende Behandlungs- und Rehabilitationsvorrichtung für degenerative Kniegelenkerkrankungen, wobei die Vorrichtung auf der Pendeltherapie bei Kniearthrose basiert, wobei die Therapie erfordert, dass ein Benutzer Beinpendelbewegungen um einen Drehpunkt des Beins des Benutzers als Behandlungs- und Rehabilitationsübung ausführt, **dadurch gekennzeichnet, dass** die Vorrichtung eine Datenerfassungskomponente für Correct and Comfortable Relationship (CCR), eine Bewegungsdaten-Erfassungskomponente, eine Stützkomponente, eine Steuerkomponente und eine Rückmeldungskomponente umfasst;

wobei die Stützkomponente dazu verwendet wird, eine Rumpfstütze und/oder eine Taillenstütze und/oder eine Ellbogenstütze für den Benutzer bereitzustellen;

die CCR-Datenerfassungskomponente ist an der Stützkomponente installiert, um während der Therapie CCR-Körperdaten des Benutzers zu erfassen; CCR bezeichnet die Beziehung zwischen korrekten und komfortablen Körperhaltungen gemäß den KOAPT-Anforderungen und Bewegungsformen des Körpers im Bewegungszustand;

die CCR-Körperdaten umfassen die Höhe des Ellbogenstützpunkts, die Entlastung des Stützbeins, den Wert des aufrechten Rumpfstatus und den Wert des aufrechten Beinstatus;

die Bewegungsdaten-Erfassungskomponente ist an der Stützkomponente installiert, um in Echtzeit Bewegungsdaten der Beinpendelbewegungen des Benutzers während der Nutzung zu erfassen; die Bewegungsdaten umfassen die Anzahl der Beinpendelbewegungen, die Bewegungsfrequenz und die Bewegungsamplitude;

die Steuerkomponente dient dazu, ein in der Steuerkomponente installiertes KOAPT-Computerprogramm auszuführen, die CCR-Körperdaten des Benutzers während der Nutzung in Echtzeit zu erfassen und in Echtzeit zu bestimmen, ob die Höhe des Ellbogenstützpunkts, die Entlastung des Stützbeins, der Wert des aufrechten Rumpfstatus und der Wert des aufrechten Beinstatus während der Übung des Benutzers mit voreingestellten CCR-Werten übereinstimmen; wenn die CCR-Körperdaten des Benutzers nicht mit den KOAPT-Anforderungen übereinstimmen, empfängt die Rückmeldungskomponente Rückmeldungsanweisungen von der Steuerkomponente und übermittelt entsprechende Informationen an den Benutzer, um die Bewegungshaltung des Benutzers zu korrigieren; das Computerprogramm empfängt außerdem in Echtzeit die von der Bewegungsdaten-Erfassungskomponente gesendeten Bewegungsdaten und führt den Benutzer an, die Behandlungs- und Rehabilitationsübung gemäß abgestuften Behandlungsmodellen durchzuführen;

die Rückmeldungskomponente dient dazu, dem Benutzer entsprechend den Rückmeldungsanweisungen der Steuerkomponente Audio- und/oder Video- und/oder taktile Signale zu senden;

die abgestuften Behandlungsmodelle bestehen aus X1, X2 und X3 Behandlungsmodellen; jedes abgestufte Behandlungsmodell weist mehrere voreingestellte Parameter auf, einschließlich: abgestufte Dauer, Einbein-Stützzahl $V$, Bewegungsamplitude $\theta$, Bewegungsfrequenz $\omega$, Beinunterstützungskraft $F$, Bewegungsmenge $C$, erforderliche Ruhezeit des Stützbeins $t$ und Entlastung des Stützbeins $\Delta a$;

die Stützkomponente umfasst einen klappbaren Beinpendelmechanismus (1), einschließlich eines Gehäuses als Bodenstütze und eines multifunktionalen Stützrahmens (2); der klappbare Beinpendelmechanismus (1) dient zur Unterstützung des Oberkörpers des Benutzers, während der multifunktionale Stützrahmen (2) zur Unterstützung der Ellbogen des Benutzers dient, und beide sind fest miteinander verbunden;

die Bewegungsdaten-Erfassungskomponente ist dazu konfiguriert, die Bewegungsamplitude mittels eines auf der oberen Oberfläche der Bodenstütze angeordneten Abstandssensors sowie eines an einer Seite des Gehäuses angeordneten fotoelektrischen Sensors zur Erkennung eines Hindernisses unterhalb des Oberschenkels des Beins des Benutzers zu erfassen; die Bewegungsamplitude $\theta$ wird berechnet als $\theta = arctan(D/(H-L))$, wobei $H$ der Abstand vom Drehpunkt des Beins des Benutzers zur oberen Oberfläche der Bodenstütze ist, $L$ der Abstand zwischen dem Abstandssensor und dem Hindernis ist und $D$ die projizierte Entfernung vom Abstandssensor zum Drehpunkt des Beins des Benutzers ist.

2. Auf KOAPT-Therapie basierende Behandlungs- und Rehabilitationsvorrichtung für degenerative Kniegelenkerkrankungen nach Anspruch 1, **dadurch gekennzeichnet, dass** das KOAPT-Computerprogramm ein Speichermodul für abgestufte Behandlungsmodelle, ein CCR-Analysemodul, ein Steuerungsmodul für den Behandlungsprozess, ein Behandlungsverfolgungsmodul, ein Speichermodul für Rückmeldungsinformationen und ein Speichermodul für Benutzerinformationen umfasst;

das Speichermodul für abgestufte Behandlungsmodelle dient zur Speicherung der abgestuften Behandlungsmodelle; das CCR-Analysemodul gibt die in Echtzeit erfassten CCR-Körperdaten in ein CCR-Analysemodell ein, bestimmt in Echtzeit die Haltungsinformationen des Benutzers und gibt CCR-Bewertungsanweisungen aus; wenn die CCR des Benutzers falsch ist, gibt es gleichzeitig ein Fehlerkennzeichen aus;

das Steuerungsmodul für den Behandlungsprozess dient dazu, basierend auf den CCR-Bewertungsanweisungen die entsprechenden Rückmeldungsanweisungen im Speichermodul für Rückmeldungsinformationen aufzurufen; das Steuerungsmodul für den Behandlungsprozess gleicht die Anzahl der Beinpendelbewegungen, die Bewegungsfrequenz und die Bewegungsamplitude, die von der Bewegungsdaten-Erfassungskomponente erfasst wurden, mit den entsprechenden Werten in den abgestuften Behandlungsmodellen ab und ruft basierend auf den Abgleichsergebnissen die entsprechenden Rückmeldungsanweisungen im Speichermodul für Rückmeldungsinformationen auf;

das Behandlungsverfolgungsmodul überwacht den Fortschritt und den Abschluss des Behandlungsprozesses des Benutzers und ruft die entsprechenden Rückmeldungsanweisungen im Speichermodul für Rückmeldungsinformationen auf;

das Speichermodul für Rückmeldungsinformationen umfasst Zählstandard-Rückmeldungsinformationen, CCR-Standard-Rückmeldungsinformationen, Anreiz-Rückmeldungsinformationen, Trennalarminformationen und Medieninformationen;

das Speichermodul für Benutzerinformationen speichert die persönlichen Informationen des Benutzers, CCR-Körperdaten und Bewegungsdaten.

3. Auf KOAPT-Therapie basierende Behandlungs- und Rehabilitationsvorrichtung für degenerative Kniegelenkerkrankungen nach Anspruch 2, **dadurch gekennzeichnet, dass** das CCR-Analysemodell durch die Gleichung $y=k1+k2+k3+k4+k5$ dargestellt wird, wobei $y$ der CCR-Anzeigewert des Benutzers ist,

$k1$ der Anzeigewert der aufrechten Rumpfhaltung ist,
$k2$ der Anzeigewert der aufrechten Beinstellung ist,
$k3$ der Anzeigewert der Entlastung des Stützbeins ist,
$k4$ der Komfort-Anzeigewert ist und
$k5$ der Redundanz-Anzeigewert ist;
wenn $y=0$, ist die CCR des Benutzers korrekt, und wenn $y=1$, ist die CCR des Benutzers falsch.

4. Auf KOAPT-Therapie basierende Behandlungs- und Rehabilitationsvorrichtung für degenerative Kniegelenkerkrankungen nach Anspruch 3, **dadurch gekennzeichnet, dass** eine Berechnungsformel für den Komfort-Anzeigewert $k4=round(\alpha Q+\beta S+\gamma T+\delta N)$ ist,

wobei $Q$ das Verhältnis der Höhe des Ellbogenstützpunkts auf beiden Seiten des Körpers zur Körpergröße ist, $S$ das Verhältnis der Entlastung des Stützbeins bei beidseitiger Unterstützung des Körpers zum Körpergewicht ist, $T$ das Verhältnis der Höhe des Ellbogenstützpunkts vor der Brust zur Körpergröße ist, $N$ das Verhältnis der Entlastung des Stützbeins bei Unterstützung an der Vorderseite der Brust zum Körpergewicht ist, und $a, \beta, \gamma, \delta$ Modellparameter sind, die durch Parameterkalibrierung erhalten und auf Grundlage der Körperdaten des Benutzers voreingestellt sind; und *"round(...)"* steht für Rundung.

5. Auf KOAPT-Therapie basierende Behandlungs- und Rehabilitationsvorrichtung für degenerative Kniegelenkerkrankungen nach Anspruch 1, **dadurch gekennzeichnet, dass** der klappbare Beinpendelmechanismus (1) ein Gehäuse (11), zwei Fußpedale (12), zwei klappbare Teleskoprahmen (13), einen Motor (14), zwei Schwenkwinkelrahmen (15) und einen Unterschenkel-Längenverstellrahmen (16) umfasst;

das Gehäuse (11) dient als Bodenstütze, wobei die zwei Fußpedale (12) auf dem Gehäuse (11) montiert sind; die zwei klappbaren Teleskoprahmen (13), die vertikal über dem Gehäuse (11) angeordnet sind, sind durch eine Gehäuseverbindung (133) fixiert; die oberen Enden der zwei Schwenkwinkelrahmen (15) sind jeweils an den klappbaren Teleskoprahmen (13) aufgehängt, um eine Hin- und Herbewegung relativ zu den klappbaren Teleskoprahmen (13) zu ermöglichen; die unteren Enden der zwei Schwenkwinkelrahmen (15) sind in den Unterschenkel-Längenverstellrahmen (16) eingesetzt und zu einer Einheit fixiert; der Motor (14) befindet sich am oberen Ende eines der Schwenkwinkelrahmen (15) und treibt die Schwenkwinkelrahmen (15) und den Unterschenkel-Längenverstellrahmen (16) zu einer Hin- und Herbewegung an;

die Steuerkomponente ist dazu konfiguriert, den Motor (14) basierend auf den abgestuften Behandlungsmodellen zu steuern und dadurch den Schwenkwinkelrahmen (15) zur Rotation anzutreiben.

6. Auf KOAPT-Therapie basierende Behandlungs- und Rehabilitationsvorrichtung für degenerative Kniegelenkerkrankungen nach Anspruch 1, **dadurch gekennzeichnet, dass** der multifunktionale Stützrahmen (2) eine Stützbasis (21), zwei Stützstangen (22) und zwei Ellbogen- und Achsel-Zweckstützhalterungen (26) umfasst; die Stützbasis (21) befindet sich am unteren Teil des multifunktionalen Stützrahmens (2), die zwei Stützstangen (22) sind symmetrisch über der Stützbasis (21) angeordnet; die Stützstangen (22) sind über einen Nockenhebel mit einer Stützeinstellstange (24) verbunden, und unterhalb der Stützeinstellstange (24) ist ein Verstärkungsflansch angeordnet; die Ellbogen- und Achsel-Zweckstützhalterungen (26) sind horizontal am oberen Teil der Stützeinstellstange (24) angeordnet.

7. Auf KOAPT-Therapie basierende Behandlungs- und Rehabilitationsvorrichtung für degenerative Kniegelenkerkrankungen nach Anspruch 5, **dadurch gekennzeichnet, dass** der Unterschenkel-Längenverstellrahmen (16) einen U-förmigen Rahmen (161), Führungsstangen (164) und ein erstes Gleitstück (165) umfasst; der Unterschenkel-Längenverstellrahmen (16) ist jeweils über zwei Enden des U-förmigen Rahmens (161) mit den zwei Schwenkwinkelrahmen (15) verbunden; eine Fußauflageplatte (165a) ist auf dem ersten Gleitstück (165) montiert und bewegt sich entlang einer Längsrichtung der Führungsstange (164) auf dem U-förmigen Rahmen (161), um ihre relative Position zum U-förmigen Rahmen (161) einzustellen, sodass sie an die Beinlänge von Benutzern unterschiedlicher Körpergröße angepasst werden kann.

8. Auf KOAPT-Therapie basierende Behandlungs- und Rehabilitationsvorrichtung für degenerative Kniegelenkerkrankungen nach Anspruch 1, **dadurch gekennzeichnet, dass** die Vorrichtung ferner einen Terminal-Stützrahmen (4) und einen Bildschirm umfasst, wobei der Terminal-Stützrahmen dazu dient, die Höhe und den Winkel des Bildschirms zu tragen und einzustellen.

9. Auf KOAPT-Therapie basierende Behandlungs- und Rehabilitationsvorrichtung für degenerative Kniegelenkerkrankungen nach Anspruch 5, **dadurch gekennzeichnet, dass** zwei klappbare Schwenksitze (10) jeweils auf den oberen Enden der zwei klappbaren Teleskoprahmen (13) installiert sind und die zwei klappbaren Schwenksitze (10) über ein Getriebegehäuse (17) und eine Kupplung (18) miteinander verbunden sind.

10. Auf KOAPT-Therapie basierende Behandlungs- und Rehabilitationsvorrichtung für degenerative Kniegelenkerkrankungen nach Anspruch 5, **dadurch gekennzeichnet, dass** ein klappbarer Schwenksitz (10) auf einem oberen Ende des klappbaren Teleskoprahmens (13) installiert ist und der klappbare Schwenksitz (10) mit dem oberen Ende des klappbaren Teleskoprahmens (13) schwenkbar verbunden ist.

11. Auf KOAPT-Therapie basierende Behandlungs- und Rehabilitationsvorrichtung für degenerative Kniegelenkerkrankungen nach Anspruch 1, **dadurch gekennzeichnet, dass** die CCR-Datenerfassungskomponente eine erste Sensorgruppe (61), eine zweite Sensorgruppe (62) und eine dritte Sensorgruppe (63) zur Erfassung von Körperdaten des Benutzers umfasst und die Bewegungsdaten-Erfassungskomponente eine vierte Sensorgruppe (64) zur Erfassung der Bewegungsdaten des Benutzers umfasst.

12. Auf KOAPT-Therapie basierende Behandlungs- und Rehabilitationsvorrichtung für degenerative Kniegelenkerkrankungen nach Anspruch 11, **dadurch gekennzeichnet, dass** die erste Sensorgruppe (61) fotoelektrische Sensoren umfasst, die vor und hinter den Fußpedalen (12) an der Stützkomponente angeordnet sind; zwei Empfangsenden der fotoelektrischen Sensoren sind jeweils vertikal über deren zwei Sendeenden angeordnet; wenn der Benutzer Beinpendelbewegungen ausführt und das Empfangsende das fotoelektrische Signal empfängt, das nicht durch den Benutzer blockiert ist, gibt die erste Sensorgruppe einen Wert von 0 für den aufrechten Rumpfstatus aus, d. h. $k1=0$; wenn das Empfangsende das fotoelektrische Signal aufgrund einer Blockierung durch den Benutzer nicht empfängt, gibt die erste Sensorgruppe einen Wert von 1 für den aufrechten Rumpfstatus aus, d. h. $k1=1$.

13. Auf KOAPT-Therapie basierende Behandlungs- und Rehabilitationsvorrichtung für degenerative Kniegelenkerkrankungen nach Anspruch 11, **dadurch gekennzeichnet, dass** die zweite Sensorgruppe (62) Verschiebungssensoren umfasst, die am Unterschenkel-Längenverstellrahmen an der Rückseite der Knie des Benutzers angeordnet sind; die Verschiebungssensoren erfassen kontinuierlich einen Anpassungszustand des Beins des Benutzers an den Unterschenkel-Längenverstellrahmen; wenn das Bein am Rahmen anliegt, gibt die zweite Sensorgruppe einen Wert von 0 für den aufrechten Status des Beins aus, d. h. $k2=0$; wenn dies nicht der Fall ist, gibt die zweite Sensorgruppe einen Wert von 1 für den aufrechten Status des Beins aus, d. h. $k2=1$.

14. Auf KOAPT-Therapie basierende Behandlungs- und Rehabilitationsvorrichtung für degenerative Kniegelenkerkrankungen nach den Ansprüchen 3 und 11, **dadurch gekennzeichnet, dass** die dritte Sensorgruppe (63) Gewichtssensoren umfasst, die unter den Fußpedalen (12) der Stützkomponente angeordnet sind, um kontinuierlich die Gewichtsdaten des Benutzers zu erfassen; die CCR-Datenerfassungskomponente ist dazu konfiguriert, den Anzeigewert der Entlastung des Stützbeins k3 basierend darauf zu berechnen, ob das Verhältnis der Entlastung des Stützbeins bei beidseitiger Unterstützung des Körpers zum Körpergewicht, kurz $S$, oder das Verhältnis der Entlastung des Stützbeins bei Unterstützung an der Vorderseite der Brust zum Körpergewicht, kurz $N$, innerhalb des Bereichs der Entlastung des Stützbeins in den vorgegebenen abgestuften Behandlungsmodellen, kurz $\Delta a$, liegt; liegt es innerhalb des Bereichs, ist die Ausgabe $k3=0$; andernfalls ist die Ausgabe $k3=1$.

15. Auf KOAPT-Therapie basierende Behandlungs- und Rehabilitationsvorrichtung für degenerative Kniegelenkerkrankungen nach Anspruch 11, **dadurch gekennzeichnet, dass** die vierte Sensorgruppe (64) den im vorderen Bereich des multifunktionalen Stützrahmens (2) angeordneten Abstandssensor und den an einer Seite des Gehäuses (11) angeordneten fotoelektrischen Sensor umfasst.

**EP 4 431 163 B1**

16. Auf KOAPT-Therapie basierende Behandlungs- und Rehabilitationsvorrichtung für degenerative Kniegelenkerkrankungen nach Anspruch 6, **dadurch gekennzeichnet, dass**, wenn der Komfort-Anzeigewert als 1 bestimmt wird, die Steuerkomponente die Rückmeldungskomponente anweist, Audio- und/oder Video- und/oder taktile Signale an den Benutzer auszugeben, um ihn/sie bei der Anpassung der Höhe einer Taillenstütze und/oder Ellbogenstütze anzuleiten, oder die Höhe der Taillenstütze und/oder Ellbogenstütze automatisch anzupassen, bis der Komfort-Anzeigewert als 0 bestimmt wird.

17. Auf KOAPT-Therapie basierende Behandlungs- und Rehabilitationsvorrichtung für degenerative Kniegelenkerkrankungen nach Anspruch 1, **dadurch gekennzeichnet, dass** die CCR-Datenerfassungskomponente und die Bewegung-Erfassungskomponente durch das integrierte Zusammenwirken verschiedener Komponenten arbeiten, einschließlich Ultraschallsensor, Verschiebungssensor, Schwerkraftsensor, fotoelektrischem Sensor, Radar und/oder Kamera.

**Revendications**

1. Dispositif de traitement et de rééducation basé sur la thérapie KOAPT pour les maladies dégénératives de l'articulation du genou, le dispositif étant conçu sur la base de la thérapie pendulaire pour l'arthrose du genou, la thérapie nécessitant qu'un utilisateur effectue des mouvements de balancement de la jambe autour d'un pivot de la jambe de l'utilisateur comme exercice de traitement et de rééducation, **caractérisé en ce que** le dispositif comprend un composant de collecte de données de relation correcte et confortable (CCR), un composant de collecte de données de quantité de mouvement, un composant de support, un composant de commande, et un composant de rétroaction ;

   dans lequel : le composant de support est utilisé pour la fourniture d'un support du tronc et/ou d'un support de la taille et/ou d'un support du coude pour l'utilisateur ;
   le composant de collecte de données CCR est installé sur le composant de support pour collecter des données corporelles CCR de l'utilisateur pendant la thérapie ; la CCR fait référence à la relation entre des postures corporelles correctes et confortables soumises aux exigences KOAPT et des formes de mouvement du corps en état de mouvement ;
   les données corporelles CCR comprennent une hauteur du point de support du coude, une réduction de charge de la jambe de support, une valeur d'état vertical du tronc et une valeur d'état vertical de la jambe ;
   le composant de collecte de données de quantité de mouvement est installé sur le composant de support pour collecter en temps réel des données de quantité de mouvement des mouvements de balancement de la jambe de l'utilisateur pendant l'utilisation ; les données de quantité de mouvement comprennent le nombre de mouvement de balancement de la jambe, la fréquence de mouvement et l'amplitude de mouvement ;
   le composant de commande est utilisé pour exécuter un programme informatique KOAPT installé dans le composant de commande, collecter en temps réel les données corporelles CCR de l'utilisateur en cours d'utilisation, et déterminer en temps réel si la hauteur du point de support du coude, la réduction de charge de la jambe de support, la valeur d'état vertical du tronc et la valeur d'état vertical de la jambe pendant l'exercice de l'utilisateur correspondent à des valeurs CCR prédéfinies ; lorsque les données corporelles CCR de l'utilisateur ne correspondent pas aux exigences KOAPT, le composant de rétroaction reçoit des instructions de rétroaction du composant de commande et communique les informations correspondantes à l'utilisateur, afin de corriger la posture de mouvement de l'utilisateur ; le programme informatique reçoit également en temps réel les données de quantité de mouvement envoyées par le composant de collecte de données de quantité de mouvement, et guide l'utilisateur pour compléter l'exercice de traitement et de rééducation selon des modèles de traitement gradués ;
   le composant de rétroaction est utilisé pour envoyer des signaux audio et/ou vidéo et/ou tactiles à l'utilisateur conformément aux instructions de rétroaction du composant de commande ;
   les modèles de traitement gradués comprennent des modèles de traitement de niveau X1, X2 et X3 ; chaque modèle de traitement gradué comporte plusieurs paramètres prédéfinis, y compris : une période graduée, un nombre de supports sur une jambe $V$, une amplitude de mouvement $\theta$, une fréquence de mouvement $\omega$, une force d'assistance de la jambe $F$, une quantité de mouvement $C$, un temps de repos nécessaire pour la jambe de support $t$, et une réduction de charge de la jambe de support $\Delta a$ ;
   le composant de support comprend un mécanisme de balancement de jambe pliable (1), comprenant un boîtier servant de support inférieur, et un cadre de support multifonctionnel (2) ; le mécanisme de balancement de jambe pliable (1) est utilisé pour supporter le torse de l'utilisateur, tandis que le cadre de support multifonctionnel (2) est utilisé pour supporter les coudes de l'utilisateur, et les deux sont fixement connectés l'un à l'autre ;

le composant de collecte de données de quantité de mouvement est configuré pour collecter l'amplitude de mouvement au moyen d'un capteur de distance placé sur la surface supérieure du support inférieur, et d'un capteur photoélectrique placé sur un côté du boîtier pour détecter un obstacle sous la cuisse de la jambe de l'utilisateur ; l'amplitude de mouvement $\theta$ est calculée comme $\theta = arctan(D/(H-L))$, dans lequel $H$ est la distance du pivot de la jambe de l'utilisateur à la surface supérieure du support inférieur, $L$ est la distance entre le capteur de distance et l'obstacle, et $D$ est la distance projetée du capteur de distance au pivot de la jambe de l'utilisateur.

2. Dispositif de traitement et de rééducation basé sur la thérapie KOAPT pour les maladies dégénératives de l'articulation du genou selon la revendication 1, **caractérisé en ce que** le programme informatique KOAPT comprend un module de stockage de modèles de traitement gradués, un module d'analyse CCR, un module de commande de processus de traitement, un module de suivi de traitement, un module de stockage d'informations de retour et un module de stockage d'informations utilisateur ;

le module de stockage de modèles de traitement gradués est utilisé pour stocker les modèles de traitement gradués ; le module d'analyse CCR saisit les données corporelles collectées en temps réel dans un modèle d'analyse CCR, détermine en temps réel les informations de posture de l'utilisateur, et exporte des instructions de jugement CCR ; lorsque la CCR de l'utilisateur est incorrect, il exporte simultanément un indicateur incorrect ; le module de commande de processus de traitement est utilisé pour appeler les instructions de rétroaction correspondantes dans le module de stockage d'informations de rétroaction sur la base des instructions de jugement CCR ; le module de commande de processus de traitement fait correspondre le nombre de mouvement de balancement de la jambe, la fréquence de mouvement et l'amplitude de mouvement collectés par le composant de collecte de données de quantité de mouvement avec les valeurs correspondantes dans les modèles de traitement gradués, et appelle les instructions de rétroaction correspondantes dans le module de stockage d'informations de rétroaction en fonction des résultats de la correspondance ;
le module de suivi de traitement surveille l'avancement et l'achèvement du processus de traitement de l'utilisateur et appelle les instructions de rétroaction correspondantes dans le module de stockage d'informations de rétroaction ;
le module de stockage d'informations de rétroaction comprend des informations de rétroaction de standard de comptage, des informations de rétroaction de standard CCR, des informations de rétroaction incitative, des informations d'alarme de séparation et des informations multimédias ;
le module de stockage d'informations utilisateur stocke les informations personnelles de l'utilisateur, les données corporelles CCR et les données de quantité de mouvement.

3. Dispositif de traitement et de rééducation basé sur la thérapie KOAPT pour les maladies dégénératives de l'articulation du genou selon la revendication 2, **caractérisé en ce que** le modèle d'analyse CCR est représenté par l'équation $y = k1 + k2 + k3 + k4 + k5$, dans laquelle $y$ est la valeur indicative CCR de l'utilisateur,

$k1$ est une valeur indicative du tronc vertical,
$k2$ est une valeur indicative de la jambe verticale,
$k3$ est une valeur indicative de réduction de charge de la jambe de support,
$k4$ est une valeur indicative de confort, et
$k5$ est une valeur indicative de redondance ;
lorsque $y = 0$, la CCR de l'utilisateur est correct, et lorsque $y = 1$, la CCR de l'utilisateur est incorrect.

4. Dispositif de traitement et de rééducation basé sur la thérapie KOAPT pour les maladies dégénératives de l'articulation du genou selon la revendication 3, **caractérisé en ce qu'**une formule de calcul pour la valeur indicative de confort est $k4 = round(\alpha Q + \beta S + \gamma T + \delta N)$,

dans lequel $Q$ est le rapport de la hauteur du point de support du coude des deux côtés du corps à la taille du corps, $S$ est le rapport de la réduction de la charge de la jambe de support lors du support des deux côtés du corps au poids du corps, $T$ est le rapport de la hauteur de point de support du coude devant la poitrine à la taille du corps, $N$ est le rapport de la réduction de la charge de la jambe de support lors du support de l'avant de la poitrine au poids du corps, et
$\alpha, \beta, \gamma, \delta$ sont des paramètres de modèle obtenus par étalonnage des paramètres, prédéfinis sur la base des données corporelles de l'utilisateur ; et « $round(...)$ » représente l'arrondi.

5. Dispositif de traitement et de rééducation basé sur la thérapie KOAPT pour les maladies dégénératives de l'articulation du genou selon la revendication 1, **caractérisé en ce que** le mécanisme de balancement de jambe pliable (1) comprend une coque (11), deux pédales (12), deux cadres télescopiques pliables (13), un moteur (14), deux cadres d'angle de balancement (15) et un cadre de réglage de la longueur de la jambe inférieure (16) ;

la coque (11) sert de support inférieur, avec les deux pédales (12) montées sur la coque (11) ; les deux cadres télescopiques pliables (13) placés verticalement au-dessus de la coque (11) sont fixés par un lien de logement (133) ; les extrémités supérieures des deux cadres d'angle de balancement (15) sont respectivement suspendues aux cadres télescopiques pliables (13) pour permettre un balancement avant et arrière par rapport aux cadres télescopiques pliables (13) ; les extrémités inférieures des deux cadres d'angle de balancement (15) sont insérées dans le cadre de réglage de la longueur de la jambe inférieure (16) et fixées en une seule unité ; le moteur (14) est situé à l'extrémité supérieure de l'un des cadres d'angle de balancement (15), entraînant les cadres d'angle de balancement (15) et le cadre de réglage de la longueur de la jambe inférieure (16) à osciller d'avant en arrière ;

le composant de commande est configuré pour commander le moteur (14) en fonction des modèles de traitement gradués, entraînant ainsi la rotation du cadre d'angle de balancement (15).

6. Dispositif de traitement et de rééducation basé sur la thérapie KOAPT pour les maladies dégénératives de l'articulation du genou selon la revendication 1, **caractérisé en ce que** le cadre de support multifonctionnel (2) comprend une base de support (21), deux tiges de support (22) et deux supports à double usage pour coude et aisselle (26) ; la base de support (21) est située au bas du cadre de support multifonctionnel (2), et les deux tiges de support (22) sont positionnées symétriquement au-dessus de la base de support (21) ; les tiges de support (22) sont connectées à une tige de réglage de support (24) via une poignée à came, et une bride de renforcement est disposée sous la tige de réglage de support (24) ; les supports à double usage pour coude et aisselle (26) sont positionnés horizontalement au sommet de la tige de réglage de support (24).

7. Dispositif de traitement et de rééducation basé sur la thérapie KOAPT pour les maladies dégénératives de l'articulation du genou selon la revendication 5, **caractérisé en ce que** le cadre de réglage de la longueur de la jambe inférieure (16) comprend un cadre en U (161), des tiges de guidage (164) et un premier curseur (165) ; le cadre de réglage de la longueur de la jambe inférieure (16) est connecté aux deux cadres d'angle de balancement (15) par les deux extrémités du cadre en U (161), respectivement ; une plate-forme de repose-pied (165a) est montée sur le premier curseur (165), et glisse le long de la direction de la longueur de la tige de guidage (164) sur le cadre en U (161) pour ajuster sa position relative au cadre en U (161), afin d'adapter la longueur de jambe des utilisateurs de différentes tailles.

8. Dispositif de traitement et de rééducation basé sur la thérapie KOAPT pour les maladies dégénératives de l'articulation du genou selon la revendication 1, **caractérisé en ce que** le dispositif comprend en outre un cadre de support terminal (4) et un écran d'affichage, le cadre de support terminal étant utilisé pour soutenir et ajuster la hauteur et l'angle de l'écran d'affichage.

9. Dispositif de traitement et de rééducation basé sur la thérapie KOAPT pour les maladies dégénératives de l'articulation du genou selon la revendication 5, **caractérisé en ce que** deux sièges pliant basculant (10) sont respectivement installés sur les sommets des deux cadres télescopiques pliables (13), et que les deux sièges pliant basculant (10) sont reliés par un boîtier d'engrenage (17) et un accouplement (18).

10. Dispositif de traitement et de rééducation basé sur la thérapie KOAPT pour les maladies dégénératives de l'articulation du genou selon la revendication 5, **caractérisé en ce qu'**un siège pliant basculant (10) est installé sur le sommet du cadre télescopique pliant (13), et le siège pliant basculant (10) est articulé avec l'extrémité supérieure du cadre télescopique pliant (13).

11. Dispositif de traitement et de rééducation basé sur la thérapie KOAPT pour les maladies dégénératives de l'articulation du genou selon la revendication 1, **caractérisé en ce que** le composant de collecte de données CCR comprend un premier groupe de capteurs (61), un deuxième groupe de capteurs (62) et un troisième groupe de capteurs (63) pour collecter les données corporelles de l'utilisateur, et le composant de collecte de données de quantité de mouvement comprend un quatrième groupe de capteurs (64) pour collecter les données de quantité de mouvement de l'utilisateur.

12. Dispositif de traitement et de rééducation basé sur la thérapie KOAPT pour les maladies dégénératives de l'articulation du genou selon la revendication 11, **caractérisé en ce que** le premier groupe de capteurs (61) comprend des capteurs photoélectriques placés devant et derrière les pédales (12) sur le composant de support ; deux extrémités réceptrices des capteurs photoélectriques sont positionnées verticalement au-dessus de leurs deux extrémités émettrices respectives ; lorsque l'utilisateur effectue des mouvements de balancement des jambes, si l'extrémité réceptrice reçoit le signal photoélectrique qui n'est pas bloqué par l'utilisateur, le premier groupe de capteurs fournit une valeur de 0 pour l'état vertical du tronc, c'est-à-dire $k$1=0 ; lorsque l'extrémité réceptrice ne reçoit pas le signal photoélectrique en raison de son blocage par l'utilisateur, le premier groupe de capteurs fournit une valeur de 1 pour l'état vertical du tronc, c'est-à-dire $k$1=1.

13. Dispositif de traitement et de rééducation basé sur la thérapie KOAPT pour les maladies dégénératives de l'articulation du genou selon la revendication 11, **caractérisé en ce que** le deuxième groupe de capteurs (62) comprend des capteurs de déplacement placés sur le cadre de réglage de la longueur de la jambe inférieure correspondant à l'arrière des genoux de l'utilisateur ; les capteurs de déplacement collectent en continu l'état d'ajustement de la jambe de l'utilisateur avec le cadre de réglage de la longueur de la jambe inférieure ; lorsque la jambe est ajustée au cadre, le deuxième groupe de capteurs fournit une valeur de 0 pour l'état vertical de la jambe, c'est-à-dire $k$2=0 ; lorsqu' ils ne sont pas en ajustement, le deuxième groupe de capteurs fournit une valeur de 1 pour l'état vertical de la jambe, c'est-à-dire $k$2=1.

14. Dispositif de traitement et de rééducation basé sur la thérapie KOAPT pour les maladies dégénératives de l'articulation du genou des revendications 3 et 11, **caractérisé en ce que** le troisième groupe de capteurs (63) comprend des capteurs de poids placés sous les pédales (12) du composant de support afin de collecter en continu les données de poids de l'utilisateur ; le composant de collecte de données CCR est configuré pour calculer la valeur indicative de réduction de charge de la jambe de support k3 en fonction de si le rapport de la réduction de la charge de la jambe de support lors du support des deux côtés du corps au poids du corps, $S$ pour faire court, ou le rapport de la réduction de la charge de la jambe de support lors du support de l'avant de la poitrine au poids du corps, $N$ pour faire court, se situe dans la plage de réduction de charge de la jambe de support dans les modèles de traitement gradués donnés, $\Delta a$ pour faire court ; si cela se situe dans la plage, la sortie est $k$3=0 ; sinon, la sortie est $k$3=1.

15. Dispositif de traitement et de rééducation basé sur la thérapie KOAPT pour les maladies dégénératives de l'articulation du genou selon la revendication 11, **caractérisé en ce que** le quatrième groupe de capteurs (64) comprend le capteur de distance positionné à l'avant du cadre de support multifonctionnel (2) et le capteur photoélectrique sur un côté du coque (11).

16. Dispositif de traitement et de rééducation basé sur la thérapie KOAPT pour les maladies dégénératives de l'articulation du genou selon la revendication 6, **caractérisé en ce que**, lorsque la valeur indicative de confort est déterminée comme étant 1, le composant de commande commande au composant de rétroaction d'émettre des signaux audio et/ou vidéo et/ou tactiles à l'utilisateur afin de le guider dans le réglage de la hauteur du support lombaire et/ou du support de coude, ou ajuste automatiquement la hauteur du support lombaire et/ou du support de coude jusqu'à ce que la valeur indicative de confort soit déterminée comme étant 0.

17. Dispositif de traitement et de rééducation basé sur la thérapie KOAPT pour les maladies dégénératives de l'articulation du genou selon la revendication 1, **caractérisé en ce que** le composant de collecte de données CCR et le composant de collecte de quantité de mouvement fonctionnent grâce au travail intégré de différentes parties, y compris le capteur ultrasonique, le capteur de déplacement, le capteur de gravité, le capteur photoélectrique, le radar et/ou la caméra.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Optical
signal
emitted by
sensors

Figure 16

Figure 17

Figure 18

Figure 19

therefore, $\tan\theta = D/(H-L)$,

$\theta = \arctan D/(H-L)$

$\theta$ is maximum movement amplitude

$L$ is the distance between the distance sensor and the obstructing object

$H$ is the distance from the user's leg pivot to the surface of the bottom support

$D$ is the projected distance from the distance sensor to the user's leg pivot

Figure 20

Figure 21

Figure 22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080234113 A1

**Non-patent literature cited in the description**

- Clinical Practice Guidelines for the Management of Osteoarthritis Pain in China (Chinese Journal of Orthopedics. 2020